(19) Europäisches Patentamt
European Patent Office
Office européen des brevets

(11) EP 4 316 517 A2

(12) **EUROPEAN PATENT APPLICATION**

(43) Date of publication:
**07.02.2024 Bulletin 2024/06**

(21) Application number: **23202447.1**

(22) Date of filing: **03.03.2017**

(51) International Patent Classification (IPC):
***A61K 39/39*** *(2006.01)*

(52) Cooperative Patent Classification (CPC):
**A61K 39/39; A61P 35/00; A61P 37/02; A61P 37/04; A61P 43/00;** A61K 2039/505; A61K 2039/55566; A61K 2039/585

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.03.2016 GB 201603805**
**08.06.2016 GB 201610018**

(62) Document number(s) of the earlier application(s) in accordance with Art. 76 EPC:
**17709038.8 / 3 423 087**

(71) Applicant: **IO Biotech ApS**
**2200 Copenhagen N (DK)**

(72) Inventor: **ANDERSEN, Mads, Hald**
**2850 Nærum (DK)**

(74) Representative: **J A Kemp LLP**
**80 Turnmill Street**
**London EC1M 5QU (GB)**

Remarks:
- •The complete document including Reference Table(s) and the Sequence Listing(s) can be downloaded from the EPO website
- •This application was filed on 09.10.2023 as a divisional application to the application mentioned under INID code 62.
- •Claims filed after the date of filing of the application / after the date of receipt of the divisional application (Rule 68(4) EPC).

(54) **COMBINATION THERAPY AGAINST CANCER**

(57) The present invention relates to a method for the prevention or treatment of cancer in a subject. The method comprises administering to said subject an immunotherapeutic composition comprising a component of an immune system checkpoint, or an immunogenic fragment of said component; and an immunomodulatory agent which blocks or inhibits an immune system checkpoint, which checkpoint may be the same as, or different from, the checkpoint of which the composition comprises a component. The invention also relates to said immunotherapeutic composition and said agent, and to kits comprising same.

EP 4 316 517 A2

## Description

### Field of the Invention

The present invention relates to a method for the prevention or treatment of cancer in a subject. The method comprises administering to said subject an immunotherapeutic composition comprising a component of an immune system checkpoint, or an immunogenic fragment of said component; and an immunomodulatory agent which blocks or inhibits an immune system checkpoint, which checkpoint may be the same as, or different from, the checkpoint of which the composition comprises a component. The invention also relates to said immunotherapeutic composition and said agent, and to kits comprising same.

### Background to the Invention

The human immune system is capable of mounting a response against cancerous tumours. Exploiting this response is increasingly seen as one of the most promising routes to treat or prevent cancer. The key effector cell of a long lasting anti-tumour immune response is the activated tumour-specific effector T cell. However, although cancer patients usually have T cells specific for tumour antigens, the activity of these T cells is frequently suppressed by inhibitory factors and pathways, and cancer remains a leading cause of premature deaths in the developed world.

Over the past decade treatments have emerged which specifically target immune system checkpoints. An example of this is Ipilimumab, which is a fully human IgG1 antibody specific for CTLA-4. Treatment of metastatic melanoma with Ipilimumab was associated with an overall response rate of 10.9% and a clinical benefit rate of nearly 30 % in a large phase III study and subsequent analyses have indicated that responses may be durable and long lasting. However, these figures still indicate that a majority of the patients do not benefit from treatment, leaving room for improvement.

Accordingly, there exists a need for methods for the prevention or treatment of cancer which augment the T cell anti-tumour response in a greater proportion of patients, but without provoking undesirable effects such as autoimmune disease.

### Summary of the Invention

The inventors have shown that an immunotherapeutic composition comprising a component of an immune system checkpoint, or an immunogenic fragment, thereof may be safely combined with administration of an additional immunomodulatory agent, providing effective treatment or prevention of cancer.

The present invention provides a method for the prevention or treatment of cancer in a subject, the method comprising administering to said subject:

(i) an immunotherapeutic composition comprising a component of an immune system checkpoint, or an immunogenic fragment of said component; and
(ii) an immunomodulatory agent which blocks or inhibits an immune system checkpoint, which checkpoint may be the same as, or different from, the checkpoint of which the composition of (i) comprises a component.

The present invention also provides said a kit comprising:

(i) An immunotherapeutic composition comprising a component of an immune system checkpoint or an immunogenic fragment thereof; and/or
(ii) An immunomodulatory agent;

optionally wherein (i) and (ii) are provided in separate sealed containers.

The present invention also provides said immunotherapeutic composition and/or said immunomodulatory agent independent of each other.

The present invention also provides a method for the prevention or treatment of cancer in a subject, the method comprising administering to said subject:

(i) an immunotherapeutic composition comprising a component of an immune system checkpoint, or an immunogenic fragment of said component; and
(ii) a composition comprising a tumour antigen or immunogenic fragment thereof.

The present invention provides an immunotherapeutic composition comprising an adjuvant and an immunogenic fragment of IDO which consists of up to 25 consecutive amino acids of the sequence of SEQ ID NO: 1, wherein said consecutive amino acids comprise the sequence of ALLEIASCL (SEQ ID NO: 2) or the sequence of DTLLKALLEIASCLEKALQVF (SEQ ID NO: 3).

**Brief Description of the Sequence Listing**

SEQ ID NO: 1 is the amino acid sequence of Indoleamine 2,3-dioxygenase (IDOl).
SEQ ID NO: 2 is the amino acid sequence of a fragment of IDO1, referred to herein as IO101 or IDO5.
SEQ ID NO: 3 is the amino acid sequence of a fragment of IDOl, referred to herein as IO102.
SEQ ID NOs 4 to 13, are the amino acid sequences of other fragments of IDOl disclosed herein.
SEQ ID NO: 14 is the amino acid sequence of PD-L1
SEQ ID NOs: 15 to 31 and 32 are the amino acid sequences of fragments of PD-L1 disclosed herein.
SEQ ID NOs: 33 and 34 are the amino acid sequences of fragments of mouse IDO1, referred to herein as IDO-Pep1 and IDO-EP2, respectively.
SEQ ID NO: 35 is a fragment of the E7 oncoprotein of HPV.

**Brief Description of the Figures**

**Figure 1: serum cytokine concentration in a patient (#10) treated in accordance with the invention.** Levels of seven different cytokines in serum are shown at several different time points during treatment. IL: Interleukin. TNF: Tumour necrosis factor. IFN: Interferon. Wk: Weeks after first series of Ipilimumab.
**Figure 2: Vaccine responses in patients treated in accordance with the invention.** Vaccine induced responses in patients were assessed with direct interferon gamma ELISpot and intracellular cytokine staining.

a) Response towards IO102 peptide. Bars represent no. of specific spots i.e. negative control subtracted.
b) 10102 reactivity in six patients treated with Ipilimumab without IDO peptide vaccine. None of these patients mounted any measurable response towards 10102.
c) Intracellular cytokine staining of 10102 stimulated T-cell cultures after four weeks of *in vitro* 10102 peptide stimulation in patients #02, #05 and #07.
d) Intracellular cytokine staining of 10102 stimulated T-cell cultures as presented in 2c, but after an additional TNF-alpha capture and rapid expansion (see methods). TNF: Tumour necrosis factor. IFN: Interferon. Wk: Weeks after first series of Ipilimumab

**Figure 3: Regulatory cells in peripheral blood of patients treated in accordance with the invention.** Flow cytometric analyses of the frequency of T cells, T helper cells, regulatory T cells (Treg) and myeloid derived suppressor cells (MDSC).

a+b) Gating strategy for Treg and MDSC (singlet gate and live cell gate was included but not shown here).
c) Percentage of Treg out of CD4+ T cells during treatment.
d) Percentage of MDSC out of live singlet PBMC.
e) Percentage of T cells in lymphocyte gate.
f) Percentage of CD4+ cells out of T cells.

**Figure 4: Clinical responses in patients treated in accordance with the invention.** Change in target lesion diameter measured according to RECIST 1.1. Patients were evaluated with PET-CT before initiation of treatment (baseline), after 12 weeks and every 8-12 weeks thereafter until progression. Change in target lesion diameter was calculated a percentage change from baseline i the sum of target lesion diameter. Lighter coloured triangles denote emergence of new lesions. PD: Progressive disease. PR: Partial response.
**Figure 5: IO102 induces superior boost of specific T cells compared to IDO5**
Flow cytometry dot-plots showing the boosting effect on the number of CMV-specific T cells of adding IDO5 or 10102 to cells stimulated with CMV peptide. Stimulation with an irrelevant HIV peptide is included as control. Results from two different PBMC batches (A and B) are shown in the figure. Percentage indicates the fraction of cells specific for CMV. NLV-PE = CMV tetramer conjugated with phycoerythrin (PE), NLV-APC = CMV tetramer conjugated with Allophycocyanin (APC)
**Figure 6: IO102 enhances the IDO SMI induced boost of specific T cells**

Flow cytometry dot-plots showing the boosting effect on the number of CMV-specific T cells of adding 10102 to cells stimulated with CMV peptide and the IDO small molecule inhibitor (SMI) 1-MT. Percentage indicates the fraction of cells specific for CMV. NLV-PE = CMV tetramer conjugated with phycoerythrin (PE), NLV-APC = CMV tetramer conjugated with Allophycocyanin (APC)

**Figure 7:** Percent lysis of THP-1 target cells induced by PBMCs (effector cells) cultured in the presence of IDO scrambled peptide (black bars) or 10102 (grey bars) at effector:target ratios as shown.

**Figure 8:** Percent lysis of THP-1 target cells induced by PBMCs (effector cells) cultured in the presence of control (IDO scrambled, black bars), IDO5 (light grey bars) or 10102 (dark grey bars) at effector:target ratios as shown.

**Figure 9:** Percent lysis of THP-1 target cells induced by PBMCs (effector cells) cultured in the presence of IDO scrambled+anti-PD-1 antibody (control) or IO102+anti-PD-1 antibody. The bars depict the lysis induced by IO102+anti-PD-1 antibody, minus control, at effector:target ratios as shown.

**Figure 10:** shows change in tumour volume (A) and percent survival (B) over time for C57BL/6 mice harbouring TC-1 tumour treated with IDO peptide (IDO-Pep1) or E7 peptide (E7-Vax). Untreated mice are shown as a control.

**Figure 11:** shows change in percent survival over time for C57BL/6 mice harbouring TC-1 tumour treated with IDO peptide (Pep1), E7 peptide (E7-Vax), or both (Pep1 + E7-Vax). Untreated mice are shown as a control.

**Figure 12:** shows change in percent survival over time for C57BL/6 mice harbouring TC-1 tumour treated with IDO peptide (Pep1), 1-MT, or both (Pep1 + 1-MT). Untreated mice are shown as a control.

**Figure 13:** shows change in tumour volume over time for BALB/c mice harbouring CT26 tumour treated with IDO peptide (EP2) or Montanide alone (Vehicle). Untreated mice are shown as a control.

## Detailed Description of the Invention

It is to be understood that different applications of the disclosed products and methods may be tailored to the specific needs in the art. It is also to be understood that the terminology used herein is for the purpose of describing particular embodiments of the invention only, and is not intended to be limiting.

In addition as used in this specification and the appended claims, the singular forms "a", "an", and "the" include plural referents unless the content clearly dictates otherwise. Thus, for example, reference to "an inhibitor" includes two or more such inhibitors, or reference to "an oligonucleotide" includes two or more such oligonucleotide and the like.

A "subject" as used herein includes any mammal, preferably a human.

A "polypeptide" is used herein in its broadest sense to refer to a compound of two or more subunit amino acids, amino acid analogs, or other peptidomimetics. The term "polypeptide" thus includes short peptide sequences and also longer polypeptides and proteins. As used herein, the term "amino acid" refers to either natural and/or unnatural or synthetic amino acids, including both D or L optical isomers, and amino acid analogs and peptidomimetics.

All publications, patents and patent applications cited herein, whether supra or infra, are hereby incorporated by reference in their entirety.

### Immune system checkpoint

Effector T cell activation is normally triggered by the T cell receptor recognising antigenic peptide presented by the MHC complex. The type and level of activation achieved is then determined by the balance between signals which stimulate and signals which inhibit the effector T cell response. The term "immune system checkpoint" is used herein to refer to any molecular interaction which alters the balance in favour of inhibition of the effector T cell response. That is, a molecular interaction which, when it occurs, negatively regulates the activation of an effector T cell. Such an interaction might be direct, such as the interaction between a ligand and a cell surface receptor which transmits an inhibitory signal into an effector T cell. Or it might be indirect, such as the blocking or inhibition of an interaction between a ligand and a cell surface receptor which would otherwise transmit an activatory signal into the effector T cell, or an interaction which promotes the upregulation of an inhibitory molecule or cell, or the depletion by an enzyme of a metabolite required by the effector T cell, or any combination thereof.

Examples of immune system checkpoints include:

a) The interaction between Indoleamine 2,3-dioxygenase (IDOl) and its substrate;
b) The interaction between PD1 and PDL1 and/or PD1 and PDL2;
c) The interaction between CTLA4 and CD86 and/or CTLA4 and CD80;
d) The interaction between B7-H3 and/or B7-H4 and their respective ligands;
e) The interaction between HVEM and BTLA;
f) The interaction between GAL9 and TIM3;
g) The interaction between MHC class I or II and LAG3; and
h) The interaction between MHC class I or II and KIR

Checkpoint (a), namely the interaction between IDOl and its substrate, is a preferred checkpoint for the purposes of the present invention. This checkpoint is the metabolic pathway in cells of the immune system requiring the essential amino acid tryptophan. A lack of tryptophan results in the general suppression of effector T cell functions and promotes the conversion of naive T cells into regulatory (i.e. immunosuppressive) T cells (Tregs). The protein IDOl is upregulated in cells of many tumours and is responsible for degrading the level of tryptophan. IDOl is an enzyme that catalyzes the conversion of L-tryptophan to N-formylkynurenine and is thus the first and rate limiting enzyme of tryptophan catabolism through the Kynurenine pathway. Therefore, IDOl is a component of an immune system checkpoint which may preferably be targeted in the method of the invention.

Another preferred checkpoint for the purposes of the present invention is checkpoint (b), namely the interaction between PD1 and either of its ligands PD-L1 and PD-L2. PD1 is expressed on effector T cells. Engagement with either ligand results in a signal which downregulates activation. The ligands are expressed by some tumours. PD-L1 in particular is expressed by many solid tumours, including melanoma. These tumours may therefore down regulate immune mediated anti-tumour effects through activation of the inhibitory PD-1 receptors on T cells. By blocking the interaction between PD1 and one or both of its ligands, a checkpoint of the immune response may be removed, leading to augmented anti-tumour T cell responses. Therefore PD1 and its ligands are examples of components of an immune system checkpoint which may preferably be targeted in the method of the invention

Another preferred checkpoint for the purposes of the present invention is checkpoint (c), namely the interaction between the T cell receptor CTLA-4 and its ligands, the B7 proteins (B7-1 and B7-2). CTLA-4 is ordinarily upregulated on the T cell surface following initial activation, and ligand binding results in a signal which inhibits further/continued activation. CTLA-4 competes for binding to the B7 proteins with the receptor CD28, which is also expressed on the T cell surface but which upregulates activation. Thus, by blocking the CTLA-4 interaction with the B7 proteins, but not the CD28 interaction with the B7 proteins, one of the normal check points of the immune response may be removed, leading to augmented anti-tumour T cell responses. Therefore CTLA4 and its ligands are examples of components of an immune system checkpoint which may preferably be targeted in the method of the invention

## Method for the prevention or treatment of cancer

The method of the invention may target any component of any checkpoint described in the preceding section.

The method of the invention concerns preventing or treating cancer.

The cancer may be prostate cancer, brain cancer, breast cancer, colorectal cancer, pancreatic cancer, ovarian cancer, lung cancer, cervical cancer, liver cancer, head/neck/throat cancer, skin cancer, bladder cancer or a hematologic cancer. The cancer may take the form of a tumour or a blood born cancer. The tumour may be solid. The tumour is typically malignant and may be metastatic. The tumour may be an adenoma, an adenocarcinoma, a blastoma, a carcinoma, a desmoid tumour, a desmopolastic small round cell tumour, an endocrine tumour, a germ cell tumour, a lymphoma, a leukaemia, a sarcoma, a Wilms tumour, a lung tumour, a colon tumour, a lymph tumour, a breast tumour or a melanoma.

Types of blastoma include hepatblastoma, glioblastoma, neuroblastoma or retinoblastoma. Types of carcinoma include colorectal carcinoma or heptacellular carcinoma, pancreatic, prostate, gastric, esophegal, cervical, and head and neck carcinomas, and adenocarcinoma. Types of sarcoma include Ewing sarcoma, osteosarcoma, rhabdomyosarcoma, or any other soft tissue sarcoma. Types of melanoma include Lentigo maligna, Lentigo maligna melanoma, Superficial spreading melanoma, Acral lentiginous melanoma, Mucosal melanoma, Nodular melanoma, Polypoid melanoma, Desmoplastic melanoma, Amelanotic melanoma, Soft-tissue melanoma, Melanoma with small nevus-like cells, Melanoma with features of a Spitz nevus and Uveal melanoma. Types of lymphoma and leukaemia include Precursor T-cell leukemia/lymphoma, acute myeloid leukaemia, chronic myeloid leukaemia, acute lymphcytic leukaemia, Follicular lymphoma, Diffuse large B cell lymphoma, Mantle cell lymphoma, chronic lymphocytic leukemia/lymphoma, MALT lymphoma, Burkitt's lymphoma, Mycosis fungoides, Peripheral T-cell lymphoma, Nodular sclerosis form of Hodgkin lymphoma, Mixed-cellularity subtype of Hodgkin lymphoma. Types of lung tumour include tumours of non-small-cell lung cancer (adenocarcinoma, squamous-cell carcinoma and large-cell carcinoma) and small-cell lung carcinoma.

The method of the invention works by activating or augmenting the T cell anti-cancer response in a subject. This is achieved by increasing cancer or tumour-specific effector T cell activation, by blocking or inhibiting one or more immune checkpoints. The method of the invention utilises at least two different approaches to block or inhibit said one or more immune checkpoints.

The first approach is to block or inhibit a checkpoint by administering an immunotherapeutic composition which results in an immune response in the subject against a component of the checkpoint, thereby blocking or inhibiting the activity of the checkpoint. Thus it may alternatively be described as a vaccine against the said component of the said checkpoint. The component of the checkpoint which is targeted by the said immune response is preferably expressed by tumour cells and may also be expressed by normal cells which have an immune inhibitory effect. Accordingly, the said immune response has a double effect in that it both blocks and inhibits the activity of the checkpoint and also directly attacks the tumour.

The second approach is to block or inhibit a checkpoint by administering an immumodulatory agent which binds to or otherwise modifies a component of the checkpoint, thereby blocking or inhibiting the activity of the checkpoint. The agent may be an antibody or small molecule inhibitor which binds to a component of the checkpoint. Multiple such agents may be administered, each of which targeting a different checkpoint or a different component of the same checkpoint.

By exploiting different approaches to block or inhibit immune system checkpoints, the method of the invention will result in a greater anti-tumour response with fewer side-effects or complications as compared to alternative methods. The anti-tumour response is typically greater than that which would be expected if only a single approach were used. In addition, there are less likely to be reductions in efficacy due to anti-drug responses, since the first approach (the vaccine) will actively benefit from such a response, which may also result in a long lasting effect. These benefits apply even if both approaches target the same immune system checkpoint.

An example of this embodiment is the use of an immunotherapeutic composition to target IDOl and as immunomodulatory agent an antibody or small molecule inhibitor of IDOl. Another example is the use of an immunotherapeutic composition to target PD-L1 and as immunomodulatory agent an antibody or small molecule inhibitor of PD1 binding to PD-L1 and/or PD-L2.

The method of the invention may also target two different immune system checkpoints, each using a different approach. In such an embodiment, the same benefits as above apply, but the anti-tumour response is also typically greater than that which would be expected if each checkpoint were targeted using the same type of approach. Examples of this embodiment include use of an immunotherapeutic composition to target IDOl (checkpoint (a)) and an antibody or small molecule inhibitor to target PD1 or CTLA4 (checkpoints (b) and (c). Other examples of this embodiment include use of an immunotherapeutic composition to target PD-L1 (checkpoint (b)) and an antibody or small molecule inhibitor to target IDOl or CTLA4 (checkpoints (a) and (c)).

In other words, the invention provides a method for the prevention or treatment of cancer in a subject, the method comprising administering to said subject:

(i) an immunotherapeutic composition comprising a component of an immune system checkpoint, or an immunogenic fragment of said component; and
(ii) an immunomodulatory agent which blocks or inhibits an immune system checkpoint, which checkpoint may be the same as, or different from, the checkpoint of which the composition of (i) comprises a component.

The present invention also provides an immunotherapeutic composition for use in a method of the invention, that is for the prevention or treatment of cancer in a subject, the immunotherapeutic composition comprising a component of an immune system checkpoint or an immunogenic fragment thereof, and the method comprising administering to said subject:

(i) said immunotherapeutic composition; and
(ii) an immunomodulatory agent which blocks or inhibits an immune system checkpoint, which checkpoint may be the same as, or different from, the checkpoint of which the composition of (i) comprises a component.

The present invention also provides an immunomodulatory agent for use in a method of the invention, that is for the prevention or treatment of cancer in a subject, wherein the immunomodulatory agent blocks or inhibits an immune system checkpoint, and the method comprising administering to said subject:

(i) said immunomodulatory agent; and
(ii) an immunotherapeutic composition comprising a component of an immune system checkpoint or an immunogenic fragment thereof, which checkpoint may be the same as, or different from, the checkpoint blocked or inhibited by (i).

Alternatively the present invention provides a method for the prevention or treatment of cancer in a subject, the method comprising administering to said subject:

(i) an immunotherapeutic composition comprising a component of an immune system checkpoint, or an immunogenic fragment of said component; and
(ii) a composition comprising a tumour antigen or immunogenic fragment thereof.

In this embodiment the method works by activating or augmenting the T cell anti-cancer response in a subject to the specific tumour antigen of the composition of (ii). This is achieved by increasing tumour-antigen-specific effector T cell activation, by administering the antigen or an immunogenic fragment thereof such that it is presented to the T cells of the subject, and simultaneously or sequentially using the immunotherapeutic composition to block or inhibit an immune checkpoint that would otherwise reduce activation of said T cells. In the context of this method, the composition comprising a tumour antigen or immunogenic fragment thereof may alternatively be described as a vaccine against the said tumour antigen, and administration with the immunotherapeutic composition of the invention may be described as potentiating the said vaccine.

The present invention also provides an immunotherapeutic composition for use in a method of the invention, that is for the prevention or treatment of cancer in a subject, the immunotherapeutic composition comprising a component of an immune system checkpoint or an immunogenic fragment thereof, and the method comprising administering to said subject:

(i) said immunotherapeutic composition; and
(ii) a composition comprising a tumour antigen or immunogenic fragment thereof.

The present invention also provides a composition comprising a tumour antigen or immunogenic fragment thereof for use in a method of the invention, that is for the prevention or treatment of cancer in a subject, wherein the method comprises administering to said subject:

(i) said composition; and
(ii) an immunotherapeutic composition comprising a component of an immune system checkpoint or an immunogenic fragment thereof.

The present invention also provides the use of an immunotherapeutic composition in the manufacture of a medicament for the prevention or treatment of cancer in a subject, the immunotherapeutic composition comprising a component of an immune system checkpoint or an immunogenic fragment thereof, which is formulated for administration before, concurrently with, and/or after an immunomodulatory agent or a composition comprising a tumour antigen or immunogenic fragment thereof.

The present invention also provides the use of an immunomodulatory agent which blocks or inhibits an immune system checkpoint in the manufacture of a medicament for the prevention or treatment of cancer in a subject, wherein the agent is formulated for administration before, concurrently with, and/or after an immunotherapeutic composition comprising a component of an immune system checkpoint or an immunogenic fragment thereof.

The present invention also provides the use of a composition comprising a tumour antigen or immunogenic fragment thereof in the manufacture of a medicament for the prevention or treatment of cancer in a subject, wherein the agent is formulated for administration before, concurrently with, and/or after an immunotherapeutic composition comprising a component of an immune system checkpoint or an immunogenic fragment thereof.

Immunotherapeutic composition

An immunotherapeutic composition of the invention results in an immune response against a component of an immune system checkpoint. The component is typically a polypeptide. Thus, the immunotherapeutic composition may comprise said component or an immunogenic fragment thereof. An "immunogenic fragment" is used herein to mean a polypeptide which is shorter than the said component of an immune system checkpoint, but which is capable of eliciting an immune response to said component.

The ability of a fragment to elicit an immune response ("immunogenicity") to a component of an immune system checkpoint may be assessed by any suitable method. Typically, the fragment will be capable of inducing proliferation and/or cytokine release *in vitro* in T cells specific for the said component, wherein said cells may be present in a sample of lymphocytes taken from a cancer patient. Proliferation and/or cytokine release may be assessed by any suitable method, including ELISA and ELISPOT. Exemplary methods are described in the Examples. Preferably, the fragment induces proliferation of component-specific T cells and/or induces the release of interferon gamma from such cells.

In order to induce proliferation and/or cytokine release in T cells specific for the said component, the fragment must be capable of binding to an MHC molecule such that it is presented to a T cell. In other words, the fragment comprises or consists of at least one MHC binding epitope of the said component. Said epitope may be an MHC Class I binding epitope or an MHC Class II binding epitope. It is particularly preferred if the fragment comprises more than one MHC binding epitope, each of which said epitopes binds to an MHC molecule expressed from a different HLA-allele, thereby increasing the breadth of coverage of subjects taken from an outbred human population.

MHC binding may be evaluated by any suitable method including the use of *in silico* methods. Preferred methods include competitive inhibition assays wherein binding is measured relative to a reference peptide. The reference peptide is typically a peptide which is known to be a strong binder for a given MHC molecule. In such an assay, a peptide is a weak binder for a given HLA molecule if it has an IC50 more than 100 fold lower than the reference peptide for the given HLA molecule. A peptide is a moderate binder is it has an IC50 more than 20 fold lower but less than a 100 fold lower than the reference peptide for the given HLA molecule. A peptide is a strong binder if it has an IC50 less than 20 fold lower than the reference peptide for the given HLA molecule.

A fragment comprising an MHC Class I epitope preferably binds to a MHC Class I HLA species selected from the group consisting of HLA-A1, HLA-A2, HLA-A3, HLA-A11 and HLA-A24, more preferably HLA-A3 or HLA-A2. Alternatively the fragment may bind to a MHC Class I HLA-B species selected from the group consisting of HLA-B7, HLA -B35, HLA -B44, HLA-B8, HLA-B15, HLA-B27 and HLA-B51.

A fragment comprising an MHC Class II epitope preferably binds to a MHC Class II HLA species selected from the group consisting of HLA-DPA-1, HLA-DPB-1, HLA-DQA1, HLA-DQB1, HLA-DRA, HLA-DRB and all alleles in these groups and HLA-DM, HLA-DO.

The immunotherapeutic composition may comprise one immunogenic fragment of a component of an immune system checkpoint, or may comprise a combination of two or more such fragments, each interacting specifically with at least one different HLA molecule so as to cover a larger proportion of the target population. Thus, as examples, the composition may contain a combination of a peptide restricted by a HLA-A molecule and a peptide restricted by a HLA-B molecule, e.g. including those HLA-A and HLA-B molecules that correspond to the prevalence of HLA phenotypes in the target population, such as e.g. HLA-A2 and HLA-B35. Additionally, the composition may comprise a peptide restricted by an HLA-C molecule.

A preferred immunotherapeutic composition of the invention results in an immune response against the polypeptide Indoleamine 2,3-dioxygenase (IDOl). In other words, the method of the invention preferably comprises administering an immunotherapeutic composition which results in an immune response against IDOl . The immunotherapeutic composition may thus alternatively be described as a vaccine against IDO1. Vaccines against IDOl which may be used as an immunotherapeutic composition of the invention are described in WO2009/143843; Andersen and Svane (2015), Oncoimmunology Vol 4, Issue 1, e983770; and Iversen et al (2014), Clin Cancer Res, Vol 20, Issue 1, p221-32. The immunotherapeutic composition of the invention may comprise IDOl or an immunogenic fragment thereof. The said fragment may consist of at least 8, preferably at least 9 consecutive amino acids of IDOl (SEQ ID NO: 1). The said fragment may consist of up to 40 consecutive amino acids of IDOl (SEQ ID NO: 1), up to 30 consecutive amino acids of IDOl (SEQ ID NO: 1), preferably up to 25 consecutive amino acids of IDOl (SEQ ID NO: 1). Thus, the fragment may comprise or consist of 8 to 40, 8 to 30, 8 to 25, 9 to 40, 9 to 30, or 9 to 25 consecutive amino acids of IDOl (SEQ ID NO: 1). The fragment preferably comprises or consists of 9 to 25 consecutive amino acids of IDOl (SEQ ID NO: 1).

The said fragment may comprise or consist of any one of the following sequences:

IO101: ALLEIASCL [199-207] (SEQ ID NO:2);
IO102: DTLLKALLEIASCLEKALQVF [194-214] (SEQ ID NO: 3);
IO×1: QLRERVEKL [54-62] (SEQ ID NO: 4);
IO×2: FLVSLLVEI [164-172] (SEQ ID NO: 5);
IO×3: TLLKALLEI [195-203] (SEQ ID NO: 6);
IO×4: FIAKHLPDL [41-49] (SEQ ID NO: 7);
IO×6: VLSKGDAGL [320-328] (SEQ ID NO:8);
IO×7: DLMNFLKTV [383-391] (SEQ ID NO: 9);
IO×8: VLLGIQQTA [275-283] (SEQ ID NO: 10);
IO×9: KVLPRNIAV [101-109] (SEQ ID NO: 11);
IO×10: KLNMLSIDHL [61-70] (SEQ ID NO: 12);
IO×11: SLRSYHLQIV [341-350] (SEQ ID NO: 13).

Numbers in square parentheses [ ] indicate the corresponding positions in the IDO polypeptide of SEQ ID NO: 1, counting from N terminus to C terminus.

The fragment preferably comprises or consists of the amino acid sequence of SEQ ID NO: 2 or SEQ ID NO: 3, and most preferably comprises of consists of the amino acid sequence of SEQ ID NO: 3. A peptide comprising or consisting of SEQ ID NO: 2 binds well to HLA-A2, which is a particularly common species of HLA. A peptide consisting of SEQ ID NO: 3 binds well to at least one of the specific class I and class II HLA species mentioned above. A fragment which comprises or consists of the amino acid sequence of SEQ ID NO: 2 or SEQ ID NO: 3 is advantageous in that it will be effective in a high proportion of the outbred human population.

Another preferred immunotherapeutic composition of the invention results in an immune response against the polypeptide, programmed death-ligand-1 (PD-L1). In other words, the method of the invention preferably comprises administering an immunotherapeutic composition which results in an immune response against PD-L1. The immunotherapeutic composition may thus alternatively be described as a vaccine against PD-L1. Vaccines against PD-L1 which may be used as an immunotherapeutic composition of the invention are described in WO2013/056716. The immunotherapeutic composition of the invention may comprise PD-L1 or an immunogenic fragment thereof. The said fragment may consist of at least 8, preferably at least 9 consecutive amino acids of PD-L1 (SEQ ID NO: 14). The said fragment may consist of up to 40 consecutive amino acids of PD-L1 (SEQ ID NO: 14), up to 30 consecutive amino acids of PD-L1 (SEQ ID NO: 14), preferably upto 25 consecutive amino acids of PD-L1 (SEQ ID NO: 14). Thus, the fragment may comprise or consist of 8 to 40, 8 to 30, 8 to 25, 9 to 40, 9 to 30, or 9 to 25 consecutive amino acids of PD-L1 (SEQ ID NO: 14). The fragment preferably comprises or consists of 9 to 25 consecutive amino acids of PD-L1 (SEQ ID NO: 14).

The said fragment may comprise or consist of any one of the following sequences:

| Name | Sequence | Start position in PDL1 (SEQ ID NO: 14) | SEQ ID NO |
|---|---|---|---|
| POL101 | LLNAFTVTV | 15 | 15 |
| POL102 | ILLCLGVAL | 247 | 16 |
| POL103 | ILGAILLCL | 243 | 17 |
| POL104 | ALQITDVKL | 98 | 18 |
| POL105 | KLFNVTSTL | 189 | 19 |
| POL106 | RLLKDQLSL | 86 | 20 |
| POL107 | QLSLGNAAL | 91 | 21 |
| POL108 | KINQRILVV | 136 | 22 |
| POL109 | HLVILGAIL | 240 | 23 |
| POL110 | RTNTTTNET | 198 | 24 |
| POL111 | CLGVALTFI | 250 | 25 |
| POL112 | QLDLAALIV | 47 | 26 |
| POL113 | SLGNAALQI | 93 | 27 |
| POL114 | VILGAILLCL | 242 | 28 |
| POL115 | HTAELVIPEL | 220 | 29 |
| POL116 | FIFMTYWHLL | 7 | 30 |
| POL117 | VIWTSSDHQV | 165 | 31 |
| IO103* | FMTYWHLLNAFTVTVPKDL | 9 | 32 |
| * Also referred to herein as PD-L1 long 1 | | | |

The said fragment preferably comprises or consists of the sequence of one of SEQ ID NOs: 15, 25, 28 or 32.

An immunotherapeutic composition may preferably comprise an adjuvant and/or a carrier. A particularly preferred immunotherapeutic composition provided by the present invention comprises and adjuvant and, as active ingredient, a polypeptide of up to 25 amino acids in length which comprises or consists of the amino acid sequence of SEQ ID NO: 3. Said composition may be provided for use in a method of the invention, or for use in any other method for the prevention or treatment of cancer which comprises administration of the composition.

Adjuvants are any substance whose admixture into the composition increases or otherwise modifies the immune response elicited by the composition. Adjuvants, broadly defined, are substances which promote immune responses. Adjuvants may also preferably have a depot effect, in that they also result in a slow and sustained release of an active agent from the administration site. A general discussion of adjuvants is provided in Goding, Monoclonal Antibodies: Principles & Practice (2nd edition, 1986) at pages 61-63.

Adjuvants may be selected from the group consisting of AlK(SO4)2, AlNa(SO4)2, AlNH4 (SO4), silica, alum, Al(OH)3, Ca3 (PO4)2, kaolin, carbon, aluminum hydroxide, muramyl dipeptides, N-acetyl-muramyl-L-threonyl-D-isoglutamine (thr-DMP), N-acetyl-nornuramyl-L-alanyl-D-isoglutamine (CGP 11687, also referred to as nor-MDP), N-acetylmuramyul-L-alanyl-D-isoglutaminyl-L-alanine-2-(1'2'-dipalmitoyl-sn -glycero-3-hydroxphosphoryloxy)-ethylamine (CGP 19835A, also referred to as MTP-PE), RIBI (MPL+TDM+CWS) in a 2% squalene/Tween-80.RTM. emulsion, lipopolysaccharides and its various derivatives, including lipid A, Freund's Complete Adjuvant (FCA), Freund's Incomplete Adjuvants, Merck Adjuvant 65, polynucleotides (for example, poly IC and poly AU acids), wax D from Mycobacterium, tuberculosis, substances found in Corynebacterium parvum, Bordetella pertussis, and members of the genus Brucella, Titermax, ISCOMS, Quil A, ALUN (see US 58767 and 5,554,372), Lipid A derivatives, choleratoxin derivatives, HSP derivatives, LPS derivatives, synthetic peptide matrixes or GMDP, Interleukin 1, Interleukin 2, Montanide ISA-51 and QS-21. Various saponin extracts have also been suggested to be useful as adjuvants in immunogenic compositions.

Granulocyte-macrophage colony stimulating factor (GM-CSF) may also be used as an adjuvant.

Preferred adjuvants to be used with the invention include oil/surfactant based adjuvants such as Montanide adjuvants (available from Seppic, Belgium), preferably Montanide ISA-51. Other preferred adjuvants are bacterial DNA based adjuvants, such as adjuvants including CpG oligonucleotide sequences. Yet other preferred adjuvants are viral dsRNA based adjuvants, such as poly I:C. GM-CSF and Imidazochinilines are also examples of preferred adjuvants.

The adjuvant is most preferably a Montanide ISA adjuvant. The Montanide ISA adjuvant is preferably Montanide ISA 51 or Montanide ISA 720.

In Goding, Monoclonal Antibodies: Principles & Practice (2nd edition, 1986) at pages 61-63 it is also noted that, when an antigen of interest is of low molecular weight, or is poorly immunogenic, coupling to an immunogenic carrier is recommended. A polypeptide or fragment of an immunotherapeutic composition of the invention may be coupled to a carrier. A carrier may be present independently of an adjuvant. The function of a carrier can be, for example, to increase the molecular weight of a polypeptide fragment in order to increase activity or immunogenicity, to confer stability, to increase the biological activity, or to increase serum half-life. Furthermore, a carrier may aid in presenting the polypeptide or fragment thereof to T-cells. Thus, in the immunogenic composition, the polypeptide or fragment thereof may be associated with a carrier such as those set out below.

The carrier may be any suitable carrier known to a person skilled in the art, for example a protein or an antigen presenting cell, such as a dendritic cell (DC). Carrier proteins include keyhole limpet hemocyanin, serum proteins such as transferrin, bovine serum albumin, human serum albumin, thyroglobulin or ovalbumin, immunoglobulins, or hormones, such as insulin or palmitic acid. Alternatively the carrier protein may be tetanus toxoid or diphtheria toxoid. Alternatively, the carrier may be a dextran such as sepharose. The carrier must be physiologically acceptable to humans and safe.

The immunotherapeutic composition may optionally comprise a pharmaceutically acceptable excipient. The excipient must be 'acceptable' in the sense of being compatible with the other ingredients of the composition and not deleterious to the recipient thereof. Auxiliary substances, such as wetting or emulsifying agents, pH buffering substances and the like, may be present in the excipient. These excipients and auxiliary substances are generally pharmaceutical agents that do not induce an immune response in the individual receiving the composition, and which may be administered without undue toxicity. Pharmaceutically acceptable excipients include, but are not limited to, liquids such as water, saline, polyethyleneglycol, hyaluronic acid, glycerol and ethanol. Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients, vehicles and auxiliary substances is available in Remington's Pharmaceutical Sciences (Mack Pub. Co., N.J. 1991).

The immunotherapeutic composition may be prepared, packaged, or sold in a form suitable for bolus administration or for continuous administration. Injectable compositions may be prepared, packaged, or sold in unit dosage form, such as in ampoules or in multi-dose containers containing a preservative. Compositions include, but are not limited to, suspensions, solutions, emulsions in oily or aqueous vehicles, pastes, and implantable sustained-release or biodegradable formulations. In one embodiment of a composition, the active ingredient is provided in dry (for e.g., a powder or granules) form for reconstitution with a suitable vehicle (e. g., sterile pyrogen-free water) prior to administration of the reconstituted composition. The composition may be prepared, packaged, or sold in the form of a sterile injectable aqueous or oily suspension or solution. This suspension or solution may be formulated according to the known art, and may comprise, in addition to the active ingredient, additional ingredients such as the adjuvants, excipients and auxiliary substances described herein. Such sterile injectable formulations may be prepared using a non-toxic parenterally-acceptable diluent or solvent, such as water or 1,3-butane diol, for example. Other acceptable diluents and solvents include, but are not limited to, Ringer's solution, isotonic sodium chloride solution, and fixed oils such as synthetic mono- or di-glycerides. Other compositions which are useful include those which comprise the active ingredient in microcrystalline form, in a liposomal preparation, or as a component of a biodegradable polymer systems. Compositions for sustained release or implantation may comprise pharmaceutically acceptable polymeric or hydrophobic materials such as an emulsion, an ion exchange resin, a sparingly soluble polymer, or a sparingly soluble salt. Alternatively, the active ingredients of the composition may be encapsulated, adsorbed to, or associated with, particulate carriers. Suitable particulate carriers include those derived from polymethyl methacrylate polymers, as well as PLG microparticles derived from poly(lactides) and poly(lactide-co-glycolides). See, e.g., Jeffery et al. (1993) Pharm. Res. 10:362-368. Other particulate systems and polymers can also be used, for example, polymers such as polylysine, polyarginine, polyomithine, spermine, spermidine, as well as conjugates of these molecules.

Immunomodulatory agent

An "immunomodulatory agent" is used herein to mean any agent which, when administered to a subject, blocks or inhibits the action of an immune system checkpoint, resulting in the upregulation of an immune effector response in the subject, typically a T cell effector response, which preferably comprises an anti-tumour T cell effector response.

The immunomodulatory agent used in the method of the present invention may block or inhibit any of the immune system checkpoints described above. The agent may be an antibody or any other suitable agent which results in said blocking or inhibition. The agent may thus be referred to generally as an inhibitor of a said checkpoint.

An "antibody" as used herein includes whole antibodies and any antigen binding fragment (i.e., "antigen-binding portion") or single chains thereof. An antibody may be a polyclonal antibody or a monoclonal antibody and may be produced by any suitable method. Examples of binding fragments encompassed within the term "antigen-binding portion" of an antibody include a Fab fragment, a F(ab')2 fragment, a Fab' fragment, a Fd fragment, a Fv fragment, a dAb fragment and an isolated complementarity determining region (CDR). Single chain antibodies such as scFv and heavy chain antibodies such as VHH and camel antibodies are also intended to be encompassed within the term "antigen-binding portion" of an antibody.

Preferred antibodies which block or inhibit the CTLA-4 interaction with B7 proteins include ipilumumab, tremelimumab, or any of the antibodies disclosed in WO2014/207063. Other molecules include polypeptides, or soluble mutant CD86 polypeptides.

Preferred antibodies which block or inhibit the PD1 interaction with PD-L1 include Nivolumab, Pembrolizumab, Lambrolizumab, Pidilzumab, and AMP-224. Anti-PD-L1 antibodies include MEDI-4736 and MPDL3280A.

Other suitable inhibitors include small molecule inhibitors (SMI), which are typically small organic molecules.

Preferred inhibitors of IDOl include Epacadostat (INCB24360), Indoximod, GDC-0919 (NLG919) and F001287. Other inhibitors of IDO1 include 1-methyltryptophan (1MT).

An immunodmodulatory agent of the invention, such as an antibody or SMI, may be formulated with a pharmaceutically acceptable excipient for administration to a subject. Suitable excipients and auxiliary substances are described above for the immunotherapeutic composition of the invention, and the same may also be used with the immunodmodulatory agent of the invention. Suitable forms for preparation, packaging and sale of the immunotherapeutic composition are also described above. The same considerations apply for the immunodmodulatory agent of the invention.

<u>Administration regimen</u>

In order to treat cancer, the immunotherapeutic composition and immunomodulatory agent are each administered to the subject in a therapeutically effective amount. By a "therapeutically effective amount" of a substance, it is meant that a given substance is administered to a subject suffering from cancer, in an amount sufficient to cure, alleviate or partially arrest the cancer or one or more of its symptoms. Such therapeutic treatment may result in a decrease in severity of disease symptoms, or an increase in frequency or duration of symptom-free periods. Such treatment may result in a reduction in the volume of a solid tumour.

In order to prevent cancer, the immunotherapeutic composition and immunodmodulatory agent are each administered to the subject in a prophylactically effective amount. By "prophylactically effective amount" of a substance, it is meant that a given substance is administered to a subject in an amount sufficient to prevent occurrence or recurrence of one or more of symptoms associated with cancer for an extended period.

Effective amounts for a given purpose and a given composition or agent will depend on the severity of the disease as well as the weight and general state of the subject, and may be readily determined by the physician.

The immunotherapeutic composition and immunomodulatory agent may be administered simultaneously or sequentially, in any order. The appropriate administration routes and doses for each may be determined by a physician, and the composition and agent formulated accordingly.

The immunotherapeutic composition is typically administered via a parenteral route, typically by injection. Administration may preferably be via a subcutaneous, intradermal, intramuscular, or intratumoral route. The injection site may be pre-treated, for example with imiquimod or a similar topical adjuvant to enhance immunogenicity. The total amount of polypeptide present as active agent in a single dose of an immunotherapeutic composition of the invention will typically be in the range of 10$\mu$g to 1000$\mu$g, preferably 10$\mu$g to 150$\mu$g.

When the immunomodulatory agent is an antibody, it is typically administered as a systemic infusion, for example intravenously. When the immunomodulatory agent is an SMI it is typically administered orally. Appropriate doses for antibodies and SMIs may be determined by a physician. Appropriate doses for antibodies are typically proportionate to the body weigh of the subject.

A typical regimen for the method of the invention will involve multiple, independent administrations of both the immunotherapeutic composition and immunodmodulatory agent. Each may be independently administered on more than one occasion, such as two, three, four, five, six, seven or more times. The immunotherapeutic composition in particular may provide an increased benefit if it is administered on more than one occasion, since repeat doses may boost the resulting immune response. Individual administrations of composition or agent may be separated by an appropriate interval determined by a physician, but the interval will typically be 1-2 weeks. The interval between administrations will typically be shorter at the beginning of a course of treatment, and will increase towards the end of a course of treatment.

An exemplary administration regimen comprises administration of an immunomodulatory agent at, for example a dose of 3 milligram per kilogram of body weight, every three weeks for a total of around four series, with an immunotherapeutic composition (typically including an adjuvant) also administered subcutaneously on the back of the arm or front of the thigh, alternating between the right and the left side. Administration of the immunotherapeutic composition may be initiated concomitantly with the first series of agent, with a total of around 7 doses of composition delivered; first weekly for a total of four and thereafter three additional doses biweekly. A regimen of this type is described in Example 1.
Another exemplary administration regimen comprises treating subjects every second week (induction) for 2.5 months and thereafter monthly (maintenance) with an immunotherapeutic composition (typically including adjuvant) administered subcutaneously. Imiquimod ointment (Aldara, Meda AS, www.meda.se) may optionally be administered 8 hours before administration of the composition and the skin covered by a patch until administration in the same area of the skin.
The invention is illustrated by the following Examples.

## Example 1 - Phase I study

### Methods

#### Study design

Patients were enrolled in a First-in-Human phase I clinical trial at the Department of Oncology at Herlev Hospital, University of Copenhagen (Herlev, Denmark) from March 2014 to August 2014. The study was originally designed as a two-armed phase I study, combining IDOl-derived peptide vaccination with standard-of-care treatment with either Ipilimumab or Vemurafenib. Only data from patients treated with Ipilimumab in combination with vaccine is reported. Subjects had to have histologically verified unresectable stage III or stage IV malignant melanoma, age $\geq$ 18 years, Eastern Cooperative Oncology Group (ECOG) performance status $\leq$ 2, at least 21 days since the last systemic treatment for melanoma and fully recovered after this and adequate haematologic, renal and liver function. A history of previous anti-CTLA-4 treatment was allowed unless treatment had been ineffective or discontinued due to toxicity.
Main exclusion criteria included concomitant systemic immunosuppressive treatment, known chronic infection, previous cancer within three years, severe concomitant medical illness, major abdominal surgery within 28 days, pregnant or lactating women, severe psychiatric illness affecting compliance, known intolerance of the vaccine adjuvants Montanide or Imiquimod, a history of autoimmunity or recipients of prophylactic vaccines within 28 days. The study was approved by The Danish Health and Medicines Agency and the local Ethics Committee, Capital Region of Denmark. The study was conducted in accordance with the Helsinki II decleration and good clinical practice (GCP). All subjects provided written informed consent before study-related procedures were performed. The study is registered at www.clinicaltrials.gov (NCT02077114) and https://eudract.ema.europa.eu/ (EudraCT# 2013-000365-37).
Primary end-point was toxicity. In addition, immune-response against the vaccine peptide and the clinical benefit of treatment were assessed. Patients received treatment with Ipilimumab, at a dose of 3 milligram per kilogram of body weight every three weeks for a total of four series. In addition to this, patients received a vaccine, containing 10102 peptide in a phosphate buffered saline (PBS)-Montanide ISA-51 emulsion, delivered subcutaneously on the back of the arm or front of the thigh, alternating between the right and the left side. Vaccination was initiated concomitantly with the first series of Ipilimumab and a total of 7 vaccines were delivered; first weekly for a total of four vaccines and thereafter three additional vaccines biweekly.

#### 10102 vaccine

The vaccine consists of a 21 amino acid peptide which corresponds to resides 194-214 of indoleamine 2,3-dioxigenase. The peptide is referred to herein as 10102. The peptide has the amino acid sequence DTLLKALLEIASCLEKALQVF (SEQ ID NO:3). It was produced to GMP standard by JPT Peptides Technologies BmbH, Berlin, Germany). For administration, the hospital pharmacy (Capital Region of Denmark) dissolved peptide in 2% dimethyl sulfoxide and 98% PBS and mixed with Montanide ISA-51 (Seppic Inc., Air Liquide Healthcare specialty ingredients, Paris La Défence, France). Topical 5% Imiqiumod Cream (Medea, Allerod, Denmark) was applied to the vaccine-site, and kept under an occlusive bandage, 6-12 hours before injection.
The peptide has numerous different HLA class I epitopes nested within the sequence, predicted to bind several of the most common HLA-alleles by *in silico* analysis (http://www.cbs.dtu.dk/services/NetMHC/).

#### Clinical Assessment Criteria

Safety of the study treatment was assessed in terms of occurrence of adverse events, graded according to Common Terminology Criteria for Adverse Events (CTCAE) version 4.0. Anti-tumour activity was evaluated with positron emission tomography-computed tomography (PET-CT) scans obtained at baseline (up to 28 days before treatment initiation) after 12 weeks and every 8-12 weeks thereafter until progression. CT scans were evaluated according to Response Evaluation Criteria In Solid Tumors (RECIST) version 1.1. Response categories are Complete Response (CR), Partial Response (PR), Progressive Disease (PD) and Stable Disease (SD). Patients receiving at least five vaccines were considered eligible for evaluation of clinical and immunological endpoints in the study.

Blood sample processing

PBMC were purified from heparinized blood using lymphoprep™(StemCell Technologies) density gradient centrifugation in LeucoSep™ tubes(Greiner Bio-One). After processing, cells were frozen in NuncR 1.8 ml CryoTube (thermo Scientific) and stored at - 150°C in 90% human AB serum (Sigma Aldrich) and 10% dimethyl sulphoxide (Herlev Hospital Pharmacy). Patient-to-processing time was sought kept as low as possible and generally processing was initiated within 4 hours. Blood for collection of serum was collected in a 8 ml VacuetteR gel-tube containing clot activator (Greiner Bio-One). Serum was aliquoted in NuncR 1.8 ml CryoTube (thermo Scientific) and stored at -80°C until analysis. PBMC were processed from 100 ml heparinized blood per sampling and serum from 8 ml full blood. Blood samples were obtained at baseline, week four, week eight and week 12. In nonprogressing patients additional blood samples were obtained concomitantly with radiological evaluations every 8-12 weeks.

*In vitro* validaton of IO102 immunogenecity

ELISpot analyses were performed in accordance with CIMT Immunoguiding Program (CIP) guidelines (http://cimt.eu/ cimt/files/dl/cip_guidelines.pdf). Vaccine-responses were assessed directly *ex vivo* in PBMC samples acquired before, during and after therapy. Cryopreserved samples were thawed and rested over night in 24 well-plates in X-vivio culture-media (Lonza) containing 5% human AB serum (Sigma). Nitrocellulose bottomed 96-well plates (MultiScreen MSIPN4W; Millipore) were coated overnight with IFN- $\gamma$ capture antibody (Mabtech). The wells were washed in PBS (Sigma-Aldrich), blocked with x-vivo for two hours at 37°C in a humidified athmosphere, and PBMC were added in a concentration of $5\times10^5$/well and $2\times10^5$/well and IO102 peptide (purchased from KJ Ross-Petersen, Klampenborg, Denmark) was added at a concentration of 5 $\mu$M. An irrelevant HIV-derived peptide derived was used as a negative control (ILKEPVHGV, purchased from KJ Ross-Petersen, Klampenborg, Denmark). After addition of peptide, the plates were allowed to incubated overnight at 37°C in a humidified atmosphere supplemented with 5% CO2. The following day medium was discarded and the wells were washed before addition of biotinylated secondary IFN-$\gamma$ antibody (Mabtech). The plates were incubated at room temperature for 2 hours, washed, and avidin-enzyme conjugate (AP-Avidin; Calbiochem/ Invitrogen Life Technologies) was added to each well. Plates were incubated at room temperature for 1 hour and the enzyme substrate NBT/BCIP (Invitrogen Life Technologies) was added to each well and incubated at room temperature for 1 to 10 minutes. Upon the emergence of dark purple spots, as judged by visual inspection, the reaction was terminated by washing de-mineralized water. The spots were counted using the ImmunoSpotSeries 2.0 Analyzer (CTL Analyzers). ELISpot responses were considered positive when the numbers of IFN-$\gamma$-secreting cells were at least 2-fold above the negative control and with a minimum of 50 spots (per $5\times10^5$ PBMC) detected. All experiments were done in triplicates. Results are presented with the average background subtracted.

Establishment of IDO-specific T cell cultures

PBMCs were stimulated with IO102 peptide in X-vivo 15 with 5% human AB serum and 120 U/ml IL2 (Novartis, Denmark) in 24 well plates (Nunc, Fischer Scientific). Initially, 20 $\mu$M peptide was used, and the cultures were restimulated every 7 days with a log10 decreased concentration of peptide for every week. Cell-cultures were supplemented with new IL2 every 7-10 days. Cells were kept at a concentration of 3-4 $\times$ $10^6$/well.
For enrichment and expansion of the IDO-specific T cells, cultured cells were restimulated, after four weeks of culture, with 20 $\mu$M peptide and enriched with the TNF- $\alpha$ Secretion Assay (Miltenyi Biotec) according to the manufacturer's instructions. Cells were peptide stimulated 2.5 hours before staining with the primary antibody. Enriched cells were expanded in a modified rapid expansion protocol (see below).

Rapid expansion protocol

T cells enriched for IDO-reactivity were expanded, and approximately 1-2 $\times$ $10^5$ cells were used initially. Cultures were started in upright T25 flasks (Nunc) containing 20 ml X-vivo 15 (Lonza) supplemented with 10 % Human AB serum, 0.6 $\mu$g anti-CD3 (OKT3, Janssen-Cilag), 6000 U/ml IL2 (Proleukin,Novartis), 1.25 $\mu$g/ml Fungizone (Squibb), 100+100 U/ml PenStrep (Gribco, Life Technologies) and 2$\times$ $10^7$ feeder-cells. As feeder-cells we used allogeneous PBMC mixed from at least three different donors. Immediately before use, feeder-cells were thawed in RPMI (Gribco, Life Technologies) with 0.025 mg/ml Pulmozyme (Roche) and γ-irradiated with 30 Gy. After 5 days of culture, 10 ml culture media was carefully aspirated and bottles were supplemented with new media containing 10 % Human AB serum, 6000 U/ml IL2, 1.25 μg/ml Fungizone and 100+100 U/ml PenStrep. Cultures were regularly assessed with regard to cell-numbers and colour of the media, and transferred to T80 or T175 flasks in addition to adding new media if appropriate. Cells were harvested after total 14 days of culture and either analyzed directly or cryopreserved in 90 human AB serum and 10 % dimethyl sulphoxide.

Intracellular cytokine staining

Cells were plated in 96-well plates (Nunc, Fischer Scientific) at a concentration of 2-3$\times 10^6$/ml. IO102 peptide was added at a concentration of 5 μM and after one hour, GolgiPlugTM (BD Biosciences) was added at a concentration of 1 μl/ml culture media. After five hours, cells were harvested and processed further. Cells were stained for surface-antigens and dead cells with the following antibodies/dyes: anti-CD3-PerCP, anti-CD4-Horizon V500, anti-CD8-FITC and LIVE/DEADR Fixable Near-IR Dead Cell Stain (Life Technologies). Cells were fixated and permeabilized using BD Cytofix/Cytoperm Kit (BD Biosciences) according to the manufacturers instructions. Cells were stained for intracellular cytokines with the following antibodies: anti-IL2-PE, anti-IFN-γ-APC (BD Biosciences) and anti-TNF-α-PE-Cy7 (Biolegend). Cells were acquired on a FACSCanto II (BD Biosciences) using FACSDiva software version 6.1.3 (BD Biosciences). FlowJo software version 10 (Tree Star, Ashland, USA) was used to determine the frequency of cytokine-positive cells.

Analyses of regulatory cells in peripheral blood

PBMC samples was thawed in 37°C RPMI 1640 medium (Lonza) supplemented with 2.5 ml DNAse containing Pulmozyme (Roche) and 0.26 mmol MgCl (Herlev Hospital Pharmacy) per 100 ml buffer. All stainings were done in phosphate buffered saline (PBS)(Lonza) containing 0.5% bovine serum albumin (Sigma-Aldrich). The following antibodies were used: FoxP3-PE, HLA-DR-HV500, CD3-PE-Cy7, CD19-PE-Cy7, CD56-PE-Cy7, CD4-HV500, CD11b-APC, CD3-APC (purchased from BD Bioscience), CD33-FITC, CD124-PE (purchased from BD Pharmigen), HELIOS-PerCP-Cy5.5, CD14-BV421, CD25-BV421 (all purchased from Biolegend), CD127-FITC, CD39-PE-Cy7 (purchased from eBioscience). Additionally, all samples were stained with the LIVE/DEADR Fixable Near-IR Dead Cell Stain Kit (life technologies). For intracellular staining of transcription factors, we used the Foxp3/Transcription Factor Staining Buffer Set (eBioscience) according to the manufacturers instructions. Cells were acquired on a FACSCanto II using FACSDiva Software version 6.1.3 (BD Biosciences). Analysis of flow data was done using FlowJo software version 10 OSX (TreeStar, Inc., Ashland, OR). Data-analysis was done after filtering of dead cells and doublets/triplets.

Cytokine Bead Array

Concentrations of interleukin (IL) 2, IL4, IL6, IL10, IL17A, TNF-α and IFN-γ in serum from patients before and during treatment was measured using the BD™ Human Th1/Th2/Th17 CBA Kit (BD Biosciences) following the manufacturer's instructions. Cytokine concentrations were measured in thawed undiluted serum-samples. Concentration of cytokines was calculated using the FCAP Array™ software version 3.0 (BD SoftFlow).

Reference patient-cohort

For comparison of clinical and immunological data, we used a cohort of patients treated with Ipilimumab at our centre (patient data etc. presented in manuscript II). These patients had participated in a biomarker-study approved by the local Ethics Committee for The Capital Region of Denmark (approval no. H-2-2012- 058) and all patients gave written informed consent. Inclusion-criteria for this protocol were similar to inclusion-criteria for the vaccine-study, though known intolerance to the vaccine adjuvants Montanide or Imiquimod was not assessed. Patients were selected as controls based on age (+/- 5 years) and M-stage. For comparison of T cell reactivity towards 10102 during Ipilimumab therapy, IFN-γ secretion was measured in a ELISpot assay as explained above. Reactivity was assessed in PBMC samples obtained before treatment-initiation and after three series of treatment.

Statistics

All statistical calculations were carried out using GraphPad Prism (GraphPad Software, La Jolla, CA). Scatter plots are presented with median (horisontal line). All tests were performed two-sided and a pvalue ≤ 0.05 was considered significant. When applicable, differences over time were assessed using Wilcoxon matched-pairs signed rank test.

## Results

Demographics

Thirteen patients were screened for inclusion in the study, 12 were included, one did not wish to participate. Two patients were diagnosed with clinically significant brain metastasis before receiving first treatment, and were excluded from the study. Ten patients received treatment in the protocol, and all were considered eligible for evaluation of clinical and immunological endpoints in the study. Patient characteristics for these ten patients are presented in table 1. Eight patients received the protocol specified four series of Ipilimumab and seven peptide vaccines. One patient developed symptomatic brain metastasis after receiving three series of Ipilimumab and five vaccines, and were excluded from the protocol because of need for high-dose corticosteroid treatment. Ipilimumab was administered as a first line therapy in all ten patients. One patient had received interferon-$\alpha$ in an adjuvant setting before entering the trial. None of the patients had received any prior systemic therapy for metastatic disease.

TABLE 1

***Patient demographics*** *- Demographics for the ten patients receiving treatment in the protocol. LN: Lymph node. M-stage: Metastatic stage at baseline according to American Joint Committee on Cancer classification.*

| Patient ID | Gender | Performance status | Previous treatment | Age at base line | dead=1 alive=0 | Number of series | Number of vaccines | M-stage |
|---|---|---|---|---|---|---|---|---|
| #01 | Male | 0 | none | 62 | 0 | 4 | 7 | M1a |
| #02 | Female | 0 | LN exairesis | 51 | 1 | 3 | 5 | M1c |
| #03 | Male | 0 | none | 46 | 0 | 4 | 7 | M1c |
| #05 | Male | 0 | LN exairesis | 29 | 0 | 4 | 7 | M1c |
| #06 | Female | 0 | none | 67 | 0 | 4 | 7 | M1b |
| #07 | Male | 0 | none | 65 | 1 | 4 | 7 | M1b |
| #09 | Male | 0 | Adjuvant interferon alpha | 65 | 0 | 4 | 7 | M1c |
| #10 | Male | 0 | none | 68 | 1 | 4 | 7 | M1a |
| #12 | Male | 0 | LN exairesis | 60 | 0 | 4 | 7 | M1a |
| #13 | Male | 0 | LN exairesis | 48 | 0 | 3 | 5 | M1c |

Toxicity

Results are presented in table 2. No CTCAE grade III or IV adverse reactions linked to the vaccine were observed. In general, treatment was well tolerated and associated with limited and manageable toxicity.

The majority of patients experienced mild to moderate injection-site reactions at the vaccine site, including itching, swelling and erythema. In most cases, symptoms responded to oral antihistamines, and symptoms remitted after completion of vaccine treatment in all patients. Interestingly, we observed increased PET-signal in many of the patients in the area of vaccine administration.

Several of the patients experienced adverse reactions most likely related to Ipilimumab treatment. Two patients experienced grade II diarrhoea, which responded to treatment with loperamide. One patient (patient #10) was admitted to our facility for treatment of grade III diarrhoea, and a colonoscopy confirmed a diagnosis of Ipilimumab induced colitis. The condition was initially treatment refractory even to high dose oral corticosteroids, but remitted after administration of intravenous methylprednisolone. One week after discharge, this patient was re-admitted at a local hospital due to relapse of diarrhoea, peripheral oedema and general malaise. At this point his laboratory investigations were remarkable for hyponatremia, hypokalemia, hypoalbuminemia and thrombocytopenia. He was treated with electrolyte and fluid replacement therapy, but died one week after admittance. At this point, the exact cause of death is not completely lucid. A PET-CT scan conducted four weeks prior to his death had revealed only cutaneous and subcutaneous metastasis and disease stabilization, and hence disease progression is not very likely. Most likely the patient had developed accelerated colitis, either due to non-adherence to treatment with corticosteroids or steroid-refractory disease. Ipilimumab induced colitis can in itself be fatal, but it is also likely that the patient developed sepsis during his hospital stay. Analyses of inflammatory cytokines in serum samples using cytokine bead array showed profoundly increased levels of IL17A and IL6 by week 12, concomitant with the development of fulminant colitis in this patient (figure 1).

Additionally, two patients experienced grade II maculopapular rash on the trunk and extremities, which responded to topical steroid treatment and regressed completely after completion of treatment. One patient experienced a flare in his pre-existing rosacea after the first dose of Ipilimumab, which regressed during subsequent therapy. One patient experienced grade II choroiditis 10 weeks after last dose of Ipilimumab, which resolved after local treatment with corticosteroids, possibly related to treatment. Finally, one patient was diagnosed with multiple clinically silent small pulmonary embolisms evident on a PET-CT scan two months after receiving the last dose of Ipilimumab. This patient had a history of lung metastasis and early-stage chronic obstructive pulmonary disease, and the event is most likely not related to either Ipilimumab or vaccine-treatment.

TABLE 2

***Adverse reactions*** - *Adverse reactions and events according to CTCAE v. 4.0. Reactions were attributed to either Ipilimumab or IDO vaccine at the treating physician's discretion.*

| Patient # | Toxicity, Ipilimumab | Toxicity, Vaccine |
|---|---|---|
| 01i | None | Erythema grade I |
| 02i | Grade I nausea | Erythema grade I, local oedema grade I, pruritus grade I |
| 03i | Grade II choroiditis | None |
| 05i | None | None |
| 06i | Pruritus grade I, Grade I diarrhoea, | Pruritus grade I, oedema grade I |
| 07i | Grade II rash, grade II diarrhoea | Erythema grade I |
| 09i | Grade II rash | Erythema grade I |
| 10i | Grade II rosacea, grade III diarrhoea | None |
| 12i | Grade II pruritus | None |
| 13i | None | Grade I pruritus, grade I erythema |

T cell response against vaccine epitopes

Spontaneous T cell responses towards 10102 (SEQ ID NO: 3) and the nested HLA-A2 epitope IDO5 (SEQ ID NO: 2, also referred to as IO101) have been reported previously, and both clinical efficacy and immunogenicity of IDO5 as a vaccine-epitope has been demonstrated in a previous clinical phase I study in lung cancer.

The present study conducted an initial screening for IFN-γ release in 10102-stimulated PBMC samples using direct ELISpot. All experiments were done in triplicates with two different cell concentrations ($5\times10^5$ and $2\times10^5$). Responses were deemed specific if the spot count were at least two-fold above the background d at least 50 spots more than the corresponding negative control (HIV peptide). As seen in figure 2a, none of the patients had detectable pretreatment responses towards I0102. However, three of the patients mounted responses exceeding the empirical threshold of 50 spots above negative control during therapy, which, to some extent, seemed to be augmented by repeated vaccinations.

Neither presence or absence of a vaccine-response or the magnitude of responses appeared to correlate with the clinical effect of treatment or type/grade of toxicity. In order to determine whether IDO-specific responses were induced by the vaccine or rather as a general phenomenon occurring during Ipilimumab treatment, IDO-reactivity in PBMC from melanoma patients treated with Ipilimumab without vaccine was measured (see methods). Reactivity before treatment and after three series was assessed. As seen in figure 2b, no induction of IDO-specific cells during Ipilimumab treatment without IDO-vaccine was observed. Hence the observed IDO-responses were induced by the vaccine. In order to further scrutinize the induced vaccine-specific T cells, cytokine-production in IO102-stimulated PBMC samples was assessed, both directly *ex vivo* and after four weeks of *in vitro* stimulation with the 10 102 peptide, using intracellular cytokine staining and flow cytometry. This was attempted in three patients, which were selected based on results from the ELISpot analyses.

As seen in figure 2c, enrichment of cytokine-positive T cells was demonstrated, primarily CD4+, upon stimulation with I0102. These cells were predominantly TNF-α producing and few of the cells secreted IFN-γ as well. Further, we conducted a purification of IDO-reactive T cells based on extracellular capture of TNF-α followed by an unspecific expansion (see methods). Results of intracellular cytokine staining after this enrichment are presented in figure 2d. As seen, CD4+ IDO reactive T cells were still present in all three patients. Additionally, enrichment of IDO reactive CD8+ T cells was observed in patient #07 and #02, though only few CD8+ T cells were present in the culture from patient #02. Neither of the subsets exhibited any significant production of IL2 (data not shown).

Dynamics of regulatory cells throughout treatment

IDO is an important mediator of immune suppression and may have important impact on the dynamics of regulatory immune cells. In mice, it has been shown that IDO-expression and metabolites produced by IDO-catalyzed degradation of tryptophan induces the generation of Tregs. Additionally, IDO may be expressed in MDSC (myeloid derived suppressor cells) and thereby represents one of several effector-mechanisms for this celltype. The levels of Tregs and MDSC in peripheral blood were measured before, during and after therapy.

Tregs were defined as CD3+CD4+CD25highCD127-FOXP3+.

MDSC were defined as PBMC negative for lineage-markers CD3, CD19 and CD56 and additionally HLADR-/lowCD14+CD11b+CD33+CD124-.

Gating strategy is presented in figures 3a+b. Results are presented in figure 3c-f.

As seen, an increased frequency of Treg cells was observed after four (two series of Ipilimumab and four vaccines) and eight weeks (three series of Ipilimumab and five vaccines). After completion of the entire treatment course by week 12, the level was still elevated compared to baseline but much less pronounced. The change was only significant at week eight ($p = 0.008$). Furthermore, Tregs were scrutinized for the expression of surface-marker CD39 and transcription-factor Helios, which may distinguish activated/non-activated and naive naturally occurring Tregs from those derived from the pool of effector T cells. No consistent change was seen in any of these subsets of Tregs (data not shown). No changes in the proportion lymphocytes positive for CD3 and only minor changes in the frequency of CD4+ T cells were observed (figure 3e+f).

With regard to MDSC, a striking mirror-image of the levels of Tregs was observed, i.e. reduced levels compared to baseline. The change, however, did not reach significance at any of the assessed time points (from baseline to week eight, $p = 0.13$). No convincing correlation between the level and the change in Tregs versus MDSC was found (data not shown).

Clinical efficacy

Of 12 included patients 10 received treatment as part of the protocol and all received at least five vaccines, as per protocol specified for evaluable patients. To this end, seven of the ten patients were still alive 10 months after end of treatment.

Results are presented in figure 4. At first evaluation, one patient (#03) had a partial remission (PR), with a 44% reduction of target lesion (TL) diameter and four patients were within the limits for stable disease (SD). Five patients progressed and were referred to other treatments. Out of these, two patients (#02 and #13) were diagnosed with brain metastasis after receiving the 3rd series of Ipilimumab and one shortly after completing the 4th series of Ipilimumab and the 7th vaccine. As brain imaging was not routinely carried out at baseline in asymptomatic patients, it is not clear whether patient #02 and #07 developed brain metastasis during therapy or if the lesions were pre-existing. However, for patient #13, a magnetic resonance scan performed immediately before inclusion had given no indications of central nervous system metastasis.

Of the four patients with SD at the first evaluation, one (#01) progressed unequivocally at the second evaluation after eight weeks with a 100% increase in the sum of TL diameter. One patient (#10) died six weeks after receiving his last treatment (see toxicity section). Two patients had continued SD. Out of these, one was treated in between evaluation-scans for a pulmonary metastasis with argon beaming, and it is not possible to determine which treatment was more responsible for the disease stabilization. Patient #03, who had an initial (unconfirmed) PR, had a further slight decrease in TL diameter of -57% compared to baseline, but unfortunately PET-CT revealed new lesions in the liver and subcutis, making his best response SD. Currently two patients have ongoing SD and are awaiting further evaluation.

In summary, the overall objective response-rate, that is complete response + partial response (CR+PR) was 0%, as none of the patients had confirmed responses better than SD. Thus, five patients (50%) were in SD by the first evaluation, out of which two were confirmed and two progressed by the 2nd evaluation, and one died between 1st and 2nd evaluation.

## Discussion

Ipilimumab, targeting the immune-inhibitory molecule CTLA-4, has been shown to prolong overall survival in patients with metastatic melanoma. This treatment may induce durable responses with dramatic reductions in tumour-load, and in some patients even complete responses. Despite this optimism, it is still a small fraction of patients who actually respond to therapy, leaving plenty of room for improvement. In an attempt to achieve this, numerous trials have focused on combination therapy, as a means of hitting more than one target at once. Several trials have tested the combination of Ipilimumab with other interventions, and so far, the most promising data has been on combination with PD-1 targeting antibody Nivolumab. However, no combination with a vaccine against IDOl has been attempted prior to the study reported here.

Treatment with the combination was associated with mild to moderate toxicity in most patients, but was generally safe and well tolerated. Most of the patients experienced some degree of local reaction at the site of vaccine-administration including erythema, oedema and non-tender lumps in the subcutaneous tissue. The latter is a common and transient side-effect to peptide vaccines containing the oil-adjuvant Montanide, which was also used in this trial.

Several of the patients experienced reactions commonly associated with Ipilimumab treatment, including diarrhoea and maculopapular rash. One patient was diagnosed with monocular choroiditis 10 weeks after the last treatment, which has previously been reported as an infrequent side-effect to Ipilimumab. Whether this was related to treatment or not is difficult to prove, given the temporal dissociation of treatment and symptoms.

One patient developed Ipilimumab associated colitis during therapy, which was initially managed by parenteral high-dose prednisolone during hospitalization at our facility. Due to unfortunate circumstances, this patient was subsequently admitted to a local hospital without experience in treating this type of patients, and died one week after admittance. At admittance, clinical chemistry and presentation indicated accelerated colitis and possible sepsis. Concomitantly with the emergence of colitis, a remarkable increase in the level of cytokines IL17A and IL6 was demonstrated in serum samples from this patient. Importantly, both cytokines may play a role in autoimmune colitis. A similar increase was not demonstrated in any of the other treated patients, indicating that the increased level of cytokines likely mediated the colitis or represent a down stream response to gut inflammation. As IDO is highly expressed in the alimentary tract, it is theoretically possible that the vaccine could cause or augment diarrhoea or colitis triggered by Ipilimumab. No association between presence or magnitude of vaccine-induced IDO-reactive T cells and the occurrence of toxicity was seen. In a previous trial conducted at the same centre, however, a minimal epitope vaccine targeting IDO in lung cancer was associated with grade I/II diarrhoea in 27 % of patients. Adverse reactions were otherwise generally manageable.

All participating patients were tested for responses against 10102 peptide using IFN-γ ELISpot, and vaccine-induced responses exceeding our empirical threshold were found in three of the ten treated patients. Responses in these patients were most likely induced by the vaccine, as none of the age and disease stage matched patients receiving Ipilimumab without vaccine mounted any signs of IDO-reactivity during therapy. None of the patients had detectable responses at baseline. The screens for IDO reactivity were carried out directly ex *vivo,* in order to gain high specificity of the assay. This disposition is, however, a trade-off of sensitivity, and it is certainly possible that an indirect approach could have demonstrated more immune-responses.

*In vitro* boosting of the IDO-response in patients demonstrating response in the initial screen was conducted in order to further characterize the reactive T cells. This revealed that reactive cells were predominantly CD4+ T cells producing TNF-$\alpha$, but after enrichment and rapid expansion, we were able to detect IDO-reactive cytotoxic T cells. IDO-reactive T cells were not readily demonstrated directly *ex vivo* by intracellular cytokine staining. Conflicting data have been published regarding the benefit of tumour-specific CD4+ T cells. Substantial evidence for the function of T helper cells in tumour-immunity indicate that the benefit is dependent on the subtype of the predominant cell and as a consequence the cytokine-milieu created in the tumour. In pre-clinical investigations, both Th1 and Th2 cells may exert anti-tumour activity either directly or via supportive and chemotactic effect on other cells, whereas Th17 and Tregs may have a negative impact depending on the tumour-type. In a vaccination-study using a long peptide derived from telomerase, CD4 T cell-responses were demonstrated with apparent cytotoxic potential and signs of clinical efficacy.

The analyses in this study revealed, that the expanded IDO-reactive CD4+ T cells were predominantly producing TNF-$\alpha$, a small fraction IFN-$\gamma$, whereas reactive CD8+ T cells were predominantly double-positive. This would suggest an inflammatory phenotype of both subsets of cells, and in the case of T helper cells, a more exhausted status possibly induced by the extensive *ex vivo* culturing of the cells. No good correlation was found between efficacy of treatment and vaccine-response as all three patients experienced disease progression. In a previous IDO-vaccination trial in lung cancer, significant correlation between pre-existing IDO-responses and treatment-response was found, but no correlation with vaccine-induced responses, which in theory could be due to migration of tumour-reactive T cells to the tumour-site upon vaccination. Several other trials have sought to correlate clinical and immune-responses yielding conflicting results, often precipitated by a low number of responders in vaccination trials.

As mentioned previously, IDO is expressed in a number of different tissues including some subtypes of MDSC, and it could be that treatment targeted against IDO might impact the frequency of MDSC. Additionally, the frequency of MDSC have been shown to be reduced by treatment with Ipilimumab. In accordance with this, decreasing frequencies of monocytic MDSC were found during treatment. Interestingly, this was mirrored by an increased level of Tregs, which could represent a counter-regulatory mechanism preventing autoimmunity, though proportionality between levels/changes in MDSC vs. Treg was not found. It has previously been reported in a number of publications that Ipilimumab may increase the frequency of Tregs in peripheral blood, though other reports have demonstrated the opposite.

Reduced levels of Tregs and no change in MDSC were observed in patients treated with Ipilimumab without the vaccine using the same gating and panel of markers as used in this study (data not shown). Assuming that the inverse changes in Tregs and MDSC is not due to random biological variation, this could indicate a specific effect of the vaccine, possibly targeting IDO expressing cells, e.g. MDSC.

At 10 months after end of treatment, seven out of ten patients are still alive, and two patients are still in stable disease. Due to the modest sample-size of the current trial, it would be expected that the number of responding patients, regardless of the vaccine, might deviate substantially from large clinical studies with Ipilimumab as a mono-treatment, solely due to biological variation. It was recently shown, that response to Ipilimumab is tightly linked to the number of non-synonymous mutations giving rise to neo-epitopes, which were present in approximately half of the scrutinized patient-samples, leaving a significant chance of an uneven distribution of this and other prognostic factors in small trials. In the tested cohort, the objective response-rate was very modest, i.e. none of of the patients had confirmed objective response. One patient had a very significant reduction in tumour-load at the 1st evaluation, but despite continued response on target lesions at the confirmatory 2nd evaluation, he progressed with new lesions at multiple sites. Likely, this patient had growth of a malignant clone less responsive or unresponsive to treatment, while genetic tumour-heterogeneity allowed some of the lesions to have continued response. Five of the ten treated patients were considered as having possible SD by the first evaluation, out of which two were confirmed at 2nd evaluation, one died and two progressed. The modest response-rate should also be seen in the light of the rather high number (three) of patients developing clinically evident brain metastasis, which invariably confers a bad prognosis.

In conclusion, administration of an IDO peptide vaccine was safe and associated with minimal toxicity in combination with Ipilimumab. The vaccine induced IDO T-cell responses which were detectable directly *ex vivo.*

**Example** 2 - **Further characterisation of IDO peptide vaccine components (1)**

*Comparison of the boost effect of IDO5 and IO102 on CMV peptide-induced stimulation of* T *cells*

Method

The overall scheme for the method is summarised in Table X.

Day 1:

- Buffy coat samples were thawed, washed twice, resuspended in medium. Cells were counted.
- Cells were then added to the wells of cell culture plates at $5\times10^6$/0.5ml in X-VIVO + 5%HS
- Wells were stimulated with CMV peptide for 2 hours at room temperature. The CMV peptide used was HCMV pp65 495-504 (NLVPMVATV).
- After 2 hours IDO5, IO102 or an irrelevant HIV peptide used as control were added according to Table X and plates incubated for additional 2 hours at room temperature.
- Added 1500 microL X-VIVO/5% HS to each well and placed plates in an incubator at 37°C.

Day 2 and 9:

- IL2 was added (120 U/ µl).

Day 8:

- Wells were restimulated with IDO5, IO102 or HIV peptides, added according to table X before incubation for 2 hours at room temperature.
- After restimulation the contents of each well were split in two and 1.5 ml X-VIVO+5%HS/well was added.
- Plates were placed in an incubator at 37°C.

Day 15:

- Cells were harvested and analysed by flow cytometry.
- The percentage of CMV-specific CD8 T cells in each well was identified by using a CD8 monoclonal antibody (mAb) as well as the tetramer complexes HLA-A2/CMV pp65 495-504.
- As control, cells were in addition stained with the tetramer complex HLA-A2/HIV-1 pol476-484 and CD8 mAb (data not shown).

**Table X**

| Well | 1 | 2 | 3 |
|---|---|---|---|
| Day | | | |
| 1 | Stim 2h CMV<br>Stim 2h HIV | Stim 2h CMV<br>Stim 2h IDO5 | Stim 2h CMV<br>Stim 2h 10102 |
| 2 | IL2 120µ/ µl | IL2 120 µ/ µl | IL2 120 µ/ µl |
| 8 | Stim 2h HIV | Stim 2h IDO-5 | Stim 2h IO102 |
| 9 | IL2 120 U/ µl | IL2 120 U/ µl | IL2 120 U/ µl |
| 15 | Tetramer staining | Tetramer staining | Tetramer staining |

Results

The results (see Figure 5) show that adding 10102 to CMV stimulated cell cultures results in induction of a larger fraction of CMV-specific T cells compared to adding IDO5/IO101. Thus 10102 is shown to be a superior inducer of specific T cells than IDO5/IO101.

**Example 3 - Further characterisation of IDO peptide vaccine components (2)**

*Test of the effect of adding IO102 in combination with the IDO small molecule inhibitor (SMI) 1-methyltryptophan (1MT) to PBMCs stimulated with CMV peptide.*

Method

The overall scheme for the method is summarised in Table Y.

Day 1:

- Buffy coat samples were thawed, washed twice, resuspended in medium. Cells were counted.
- Cells were then added to the wells of cell culture plates at $5\times10^6$/2ml in X-VIVO + 5%HS
- Wells were stimulated with peptides and SMI as shown in Table Y. The CMV peptide used was HCMV pp65 495-504 (NLVPMVATV).
- Plates were placed in an incubator at 37 degrees.

Day 2 and 9:

- IL2 was added (100 U/ μl).

Day 8:

- 1ml was removed from each well and 1ml fresh medium added.
- Wells were restimulated with peptides and SMI according to table Y
- Plates were placed in an incubator at 37°C.

Day 15:

- Cells were harvested and analysed by flow cytometry.
- The percentage of CMV-specific CD8 T cells in each well was identified by using a CD8 monoclonal antibody (mAb) as well as the tetramer complexes HLA-A2/CMV pp65 495-504.
- As control, cells were in addition stained with the tetramer complex HLA-A2/HIV-1 pol476-484 and CD8 mAb (data not shown).

**Table Y**

| Well | 1 | 2 | 3 |
|---|---|---|---|
| Day | | | |
| 1 | CMV 1 μM<br>HIV 1 μM | CMV 1μM<br>1MT-DL 10μM | CMV 1μM<br>IDO-Long 1μM<br>1MT-DL 10μM |
| 2 | IL2 100 U/μl | IL2 100 U/μl | IL2 100 U/μl |
| 8 | CMV 1μM<br>HIV 1μM | CMV 1 μM<br>1MT-DL 10μM | CMV 1 μM<br>IDO-Long 1μM<br>1MT-DL 10μM |

(continued)

| Well | 1 | 2 | 3 |
|---|---|---|---|
| Day | | | |
| 9 | IL2 100 U/μl | IL2 100 U/μl | IL2 100 U/μl |
| 15 | Tetramer staining | Tetramer staining | Tetramer staining |

Results

The results (see Figure 6) show that boosting CMV-specific T cell stimulation with IO102 in combination with the IDO small molecule inhibitor 1MT is more potent than 1MT alone. Thus simultaneous targeting of IDO using both 10102 and SMI is more effective that mono-targeting using IDO inhibitors such as 1MT. 10102 enhances the IDO SMI induced boost of specific T cells

**Example 4 - Further characterisation of IDO peptide vaccine components (3)**

*Adding IO102, but not a peptide containing the same amino acids as IO102 in scrambled sequence, enhanced the allogenic killing of the acute monocytic leukemia cell line THP-1 by PBMCs.*

Method

The overall scheme for the method is summarised in Table Z4.

Buffycoats were thawed (day 0), washed twice, resuspended in medium, counted and plated into wells as set out in Table Z4. THP-1 cells were irradiated with 30 GY and washed twice in medium. THP-1 cells were counted and added to PBMC containing wells as set out in Table Z4 (total volume 2 ml). Plates were placed in an incubator at 37 degrees. Peptides and cytokines were added at the intervals and concentrations shown in Table Z5. On days 7 and 14, 1 ml supernatant/well was removed. Fresh irradiated THP-1 cells were prepared and counted as above and 1ml added to the wells as shown in Table Z4. Cells were harvested and analyzed for cytotoxic activity on day 17. A standard $^{51}$Cr-release assay was used to measure the cytotoxic potential of the peptide treated PBMCs. $^{51}$Cr labeled THP-1 cells were used as target cells and peptide treated PBMCs as effector cells. Triton-X treated wells was used as maximum measurable lysis and medium alone as minimum lysis.

$$\% \text{ specific lysis} = ((cpm_{sample} - cpm_{minimum})/(cpm_{maximum} - cpm_{minimum})) \times 100\%$$

**Table Z4**

| Day | IO102 | IDO scrambled |
|---|---|---|
| 0 | 2x10e6 PBMC + 2x10e5 irr. THP-1<br>IL7, 40 U/ml | |
| 1 | IL12 20 U/ml | |
| 3 | IO102, 0.1 μM | IDO scrambled, 0.1 μM |
| 4 | IL2 120 U/μl | |
| 7 | + 2x10e5 irr THP-1 | |
| 10 | IO102, 0.1 μM | IDO scrambled, 0.1 μM |
| 11 | IL2 120 U/μl | |
| 14 | + 2x10e5 irr THP-1 | |
| 17 | Cytotoxicity assay | |

Results

Adding 10102 to a culture of PMBCs and THP-1 cancer cells enhanced the allogenic killing of the THP-1 cells. The boosted cytotoxicity is specific for the 10102 peptide as a peptide containing the same amino acids as 10102 but in a scrambled sequence (CILDSKLEVEALAQLLTFALK (SEQ ID NO: 15), Figure 7, black bars) induced less lysis of THP-1 cells at a range of different effector target ratios (E/T) compared to IO102 (Figure 7, grey bars).

**Example 5 - Further characterisation of IDO peptide vaccine components (4)**

*IO102 induces better PBMC mediated cytotoxicity of THP-1 cells compared to a peptide containing the same amino acids as IO102 in scrambled sequence (control, IDO scrambled). Also IO102 results in better killing of THP-1 cells at low effector-target (E/T) ratios compared to IDO5.*

Method

The overall scheme for the method is summarised in Table Z5.

Buffycoats were thawed (day 0), washed twice, resuspended in medium, counted and plated into wells as set out in Table Z5. THP-1 cells were irradiated with 30 GY and washed twice in medium. THP-1 cells were counted and added to PBMC containing wells as set out in Table Z5 (total volume 2 ml). Plates were placed in an incubator at 37 degrees. Peptides and cytokines were added at the intervals and concentrations shown in Table Z5. On day 7, 1 ml supernatant/well was removed. Fresh irradiated THP-1 cells were prepared and counted as above and 1ml added to the wells as shown in Table Z5 Cells were harvested and analyzed for cytotoxic activity on day 23. A standard $^{51}$Cr-release assay was used to measure the cytotoxic potential of the peptide treated PBMCs. $^{51}$Cr labeled THP-1 cells were used as target cells and peptide treated PBMCs as effector cells. Triton-X treated wells was used as maximum measurable lysis and medium alone as minimum lysis.

$$\% \text{ specific lysis} = ((cpm_{sample} - cpm_{minimum})/(cpm_{maximum} - cpm_{minimum})) \times 100\%$$

**Table Z5**

| Day | Control (IDO scrambled) | IDO5 | IO102 |
|---|---|---|---|
| 0 | 2x10e6 PBMC + 2x10e5 irr. THP-1<br>IL7, 40 U/ml | | |
| 1 | IL12 20 U/ml | | |
| 2 | IDO scrambled, 0.1 μM | IDO5, 0.1 μM | IO102, 0.1 μM |
| 3 | IL2 120 U/ul | | |
| 7 | + 2x10e5 irr THP-1 | | |
| 9 | IDO scrambled, 0.1 μM | IDO5, 0.1 μM | IO102, 0.1 μM |
| 10 | IL2 120 U/ul | | |
| 16 | IDO scrambled, 0.1 μM | IDO5, 0.1 μM | IO102, 0.1 μM |
| 17 | IL2 120 U/ul | | |
| 23 | Cytotoxicity assay | | |

Results

Adding 10102 (Figure 8, dark grey bars) to a culture of PMBCs and THP-1 cancer cells enhanced the allogenic killing of the THP-1 cells compared to the control peptide, IDO scrambled (Figure 8, black bars) at all E/T ratios tested (2:1 - 60:1). In addition, adding 10102 to the PBMC culture induces more efficient lysis of THP-1 cells compared to adding IDO5 peptide (Figure 8, light grey bars), at low E/T ratios. This indicated that 10102 specific T cells more efficiently support an allogenic anti-cancer T-cell response.

**Example 6 - characterisation of IDO peptide vaccine in combination with an additional checkpoint inhibitor**

*IO102 + anti-PD1 induces better PBMC mediated cytotoxicity of THP-1 cells compared to control peptide +anti-PD-1.*

The overall scheme for the method is summarised in Table Z6.

Buffycoats were thawed (day 0), washed twice, resuspended in medium, counted and plated into wells as set out in Table Z6. THP-1 cells were irradiated with 30 GY and washed twice in medium. THP-1 cells were counted and added to PBMC containing wells as set out in Table Z5 (total volume 2 ml). Plates were placed in an incubator at 37 degrees. Peptides, antibody (pembrolizumab, Merck) and cytokines were added at the intervals and concentrations shown in Table Z6. On day 7, 1 ml supernatant/well was removed. Fresh irradiated THP-1 cells were prepared and counted as above and 1ml added to the wells as shown in Table Z6. Cells were harvested and analyzed for cytotoxic activity on day 23. A standard $^{51}$Cr-release assay was used to measure the cytotoxic potential of the peptide treated PBMCs. $^{51}$Cr labeled THP-1 cells were used as target cells and peptide treated PBMCs as effector cells. Triton-X treated wells was used as maximum measurable lysis and medium alone as minimum lysis.

$$\% \text{ specific lysis} = ((cpm_{sample} - cpm_{minimum})/(cpm_{maximum} - cpm_{minimum})) \times 100\%$$

**Table Z6**

| Day | IO102 + anti-PD1 | IDO scrambled + anti-PD1 |
|---|---|---|
| 0 | 2x10e6 PBMC + 2x10e5 irr. THP-1<br>IL7, 40 U/ml | |
| 1 | IL12 20 U/ml | |
| 2 | IO102, 1 μM | IDO scrambled, 1 μM |
| 3 | IL2 120 U/ul | |
| 7 | + 2x10e5 irr THP-1 | |
| 9 | IO102, 1 μM* + anti-PD1, 0.5 μg | IDO scrambled, 1 μM + anti-PD1, 0.5 μg |
| 10 | IL2 120 U/ul | |
| 16 | IO102, 1 μM + anti-PD1, 0.5 μg | IDO scrambled, 1 μM + anti-PD1, 0.51 μg |
| 17 | IL2 120 U/ul | |
| 23 | Cytotoxicity assay | |

**Results**

Adding a combination of 10102 and anti-PD-1 antibody to a culture of PMBCs enhanced the allogenic killing of THP-1 target cells, as compared to the lysis by PBMCs cultured with a combination of IDO scrambled control peptide and anti-PD-1 antibody (Figure 9) at all E/T ratios (0.7:1 - 20:1).

**Example 7 -testing of mouse model for IDO peptide vaccines**

C57BL/6 mice harbouring TC-1 tumour were treated with TC-1 specific E7-peptide (RAHYNIVTF; SEQ ID NO: 35) or IDO-Pep1 (MTYENMDIL; SEQ ID NO: 33) and the efficacy of the peptide vaccines was assessed by tumour burden and survival. The mouse IDO peptide sequence (IDO-Pep1) was selected to provide a murine analog for the human IDO peptides tested in the earlier Examples, because the sequence of murine IDOI differs to that of human IDO1.

Method

4-6 week-old C57BL/6 mice were injected with 70,000 cell/ mouse of TC-1 cells. Treatment in respective groups was started when tumour reached an average size of approximately 0.075 cm3 (approx. 10 days after tumour inoculation). Peptides used for vaccination included TC1-specific E7-peptide (RAHYNIVTF; SEQ ID NO: 35) and IDO-Pep1 (MTYENMDIL; SEQ ID NO: 33). The mouse IDO peptide was designed based on MHC class I and II binding algorithm. Mice were vaccinated subcutaneously with respective peptides at a dosage of 100μg/mouse administered in combination with a Pan-HLADR-binding epitope (PADRE; aK-Cha-VAAWTLKAAa, 20μg/mouse) and QuilA (10μg/mouse). Mice were vaccinated two-three times with one-week interval, and the efficacy of the peptide vaccines was assessed by measuring tumour volume and/or overall survival.

Results

Vaccination with mouse IDO peptide demonstrated anti-tumour protective effect in TC-1 tumour model, providing greater protection than when vaccinated with tumour-specific peptide antigen, E7 peptide. See Figure 10.

**Example 8 - testing IDO peptide vaccine in a mouse model in combination with a tumour antigen vaccine**

C57BL/6 mice harbouring TC-1 tumour were treated with TC-1 specific E7-peptide (RAHYNIVTF; SEQ ID NO: 35), or IDO-Pep1 (MTYENMDIL; SEQ ID NO: 33), or a combination of both E7 and IDO-Pep1. The efficacy of the vaccines was assessed by overall survival. The Method was the same as for Example 7, except for the inclusion of the peptide combination.

Results

Vaccinating mice with E7 peptide has therapeutic efficacy in TC-1 model, as expected (and shown in figure 10). However when E7 peptide was administered together with IDO-Pep1 it provides even greater protection than E7 or IDO-peptide alone. See Figure 11.

**Example 9 - testing IDO peptide vaccine in a mouse model in combination with additional checkpoint inhibitor**

C57BL/6 mice harbouring TC-1 tumour were treated with IDO-Pep1 (MTYENMDIL; SEQ ID NO: 33) alone, 1-methyl tryptophan (1-MT) alone, or a combination of both IDO-Pep1 and 1-MT. The efficacy of the vaccines was assessed by overall survival.

Method

4-6 week-old C57BL/6 mice were injected with 70,000 cell/ mouse of TC-1 cells. Treatment in respective groups was started when tumour reached an average size of approximately 0.075 $cm^3$ (approx. 10 days after tumour inoculation). For vaccination mouse IDO epitope, IDO-Pep1 was used. Mice were vaccinated subcutaneously with IDO Pep-1 at a dosage of 100μg/mouse in combination with a Pan-HLADR-binding epitope (PADRE; aK-Cha-VAAWTLKAAa, 20μg/mouse) and QuilA (10μg/mouse). Mice were vaccinated two-three times with one-week interval, and the efficacy of the peptide vaccines was assessed by measuring tumour volume and/or survival. A group of mice were treated with 1-methyl tryptophan (1-MT) given in drinking water (2mg/ml) for the duration of the study, and additional group of mice were treated with both IDO-Pep1 and 1-MT.

Results

A modest therapeutic efficacy is achieved when TC-1 harbouring mice were treated with oral a-MT. The efficacy was enhanced when these mice were co-administered with IDO Pep-1, as demonstrated by prolonged survival. See Figure 12.

**Example 10 -testing of alternative mouse model for IDO peptide vaccines**

BALB/c mice were prophylactically vaccinated with a mouse IDO peptide IDO-EP2 (LPTLSTDGL; SEQ ID NO: 34) and challenged with CT26 tumours 7 days later. The efficacy of the peptide vaccine was assessed by tumour burden. The mouse IDO peptide sequence (IDO-EP2) was selected to provide a murine analog for the human IDO peptides tested in the earlier Examples, because the sequence of murine IDOl differs to that of human IDOl .

Method

6-10 week-old BALB/c mice were immunized by subcutaneous injection at the base of the tail of 100 μg peptide IDO-EP2 + 30 μg CpG in 100 μL Montanide adjuvant [Montanide ISA 51 VG (Seppic)] prepared as a 1:1 emulsification. Montanide alone (Vehicle) was used as a control. Some mice were left untreated as a further control. Immunizations were carried out 7 days prior to tumour challenge. For CT26 tumour cell engraftment, each mouse received two subcutaneous injections (one on each flank) of $1\times10^5$ CT26 in 100 μL serum free DMEM. Caliper measurements of tumour length and width were recorded every 3-4 days beginning at day 6. Tumour volume was calculated using the formula $0.52(L \times W^2)$. Mice were euthanized when the combined tumour volume reached the defined endpoint of approximately 2,000 $mm^3$.

Results

Prophylactic vaccination with mouse IDO peptide demonstrated anti-tumour protective effect in CT26 tumour model. See Figure 13.

**Example 11 - clinical trial of PD-L1/IDO peptide vaccine combined with anti-PD1 antibody**

The primary objective is to assess tolerability and safety of a peptide vaccine containing the peptides IDO long (DTLLKALLEIASCLEKALQVF; SEQ ID NO: 3) and PD-L1 long1 (FMTYWHLLNAFTVTVPKDL; SEQ ID NO: 32) with Montanide ISA 51 as adjuvant, when administered to patients with metastatic malignant melanoma (MM) in combination with the immune checkpoint blocking antibody Nivolumab (specific for human PD1). The endpoint is adverse events (AE) assessed by CTCAE 4.0.

The secondary objective is to evaluate the immune response before, during and after treatment. Blood samples will be taken before treatment and thereafter every third month up to 5 years. Antigen specific immune reactivity will be tested by use of a panel of relevant immunological assays including ELISPOT, proliferation assays, cytotoxic assays, intracellular staining (ICS), and multimeric staining of PD-L1 and IDO specific CD8 T cells. Efforts shall be made to take biopsies from the available tumor lesions or involved lymph nodes before the 1st vaccine and after the 6th vaccine. The objective is to evaluate the tumor immune microenvironment of each patient. Immunohistochemistry, gene expression quantification on different immune genes and whole exome sequencing to assess initial mutational status will be conducted

The tertiary objective is to evaluate the clinical efficacy of the treatment. The endpoints will be objective response (OR), progression free survival (PFS) and overall survival (OS)

The study is designed as an open phase I/II trial. In phase I, 6 patients with MM will be treated. Before phase II can start, all 6 patients must receive the first 4 treatments without any grade 3-4 adverse events, other than those expected with Nivolumab.

If 3 or more patients experience grade 3-4 AE in phase I associated with the vaccination, the trial will be stopped. In phase II, additionally 26 patients will be included.

Patients included in the trial will be treated with Nivolumab in accordance with the standard regimen, which at the moment involves outpatient IV infusions of 3 mg/kg every second week as long as there is a clinical effect. The PD-L1/IDO vaccine is given from the start of Nivolumab and every second week for the first 6 vaccines and thereafter every fourth week up 47 weeks. 15 vaccines will be given in total. At the end of vaccination, patients who are not excluded from the protocol because of progression will continue treatment with Nivolumab in accordance with the usual guidelines.

**FULL LENGTH PROTEIN SEQUENCES**

SEQ ID NO: 1 (IDO1)

```
Met Ala His Ala Met Glu Asn Ser Trp Thr Ile Ser Lys Glu Tyr His
1               5                   10                  15
Ile Asp Glu Glu Val Gly Phe Ala Leu Pro Asn Pro Gln Glu Asn Leu
            20                  25                  30
Pro Asp Phe Tyr Asn Asp Trp Met Phe Ile Ala Lys His Leu Pro Asp
        35                  40                  45
Leu Ile Glu Ser Gly Gln Leu Arg Glu Arg Val Glu Lys Leu Asn Met
    50                  55                  60
Leu Ser Ile Asp His Leu Thr Asp His Lys Ser Gln Arg Leu Ala Arg
65                  70                  75                  80
Leu Val Leu Gly Cys Ile Thr Met Ala Tyr Val Trp Gly Lys Gly His
                85                  90                  95
Gly Asp Val Arg Lys Val Leu Pro Arg Asn Ile Ala Val Pro Tyr Cys
            100                 105                 110
Gln Leu Ser Lys Lys Leu Glu Leu Pro Pro Ile Leu Val Tyr Ala Asp
        115                 120                 125
Cys Val Leu Ala Asn Trp Lys Lys Lys Asp Pro Asn Lys Pro Leu Thr
    130                 135                 140
Tyr Glu Asn Met Asp Val Leu Phe Ser Phe Arg Asp Gly Asp Cys Ser
145                 150                 155                 160
Lys Gly Phe Phe Leu Val Ser Leu Leu Val Glu Ile Ala Ala Ala Ser
                165                 170                 175
Ala Ile Lys Val Ile Pro Thr Val Phe Lys Ala Met Gln Met Gln Glu
            180                 185                 190
Arg Asp Thr Leu Leu Lys Ala Leu Leu Glu Ile Ala Ser Cys Leu Glu
        195                 200                 205
Lys Ala Leu Gln Val Phe His Gln Ile His Asp His Val Asn Pro Lys


    210                 215                 220
Ala Phe Phe Ser Val Leu Arg Ile Tyr Leu Ser Gly Trp Lys Gly Asn
225                 230                 235                 240
Pro Gln Leu Ser Asp Gly Leu Val Tyr Glu Gly Phe Trp Glu Asp Pro
                245                 250                 255
Lys Glu Phe Ala Gly Gly Ser Ala Gly Gln Ser Ser Val Phe Gln Cys
            260                 265                 270
Phe Asp Val Leu Leu Gly Ile Gln Gln Thr Ala Gly Gly Gly His Ala
        275                 280                 285
Ala Gln Phe Leu Gln Asp Met Arg Arg Tyr Met Pro Pro Ala His Arg
    290                 295                 300
Asn Phe Leu Cys Ser Leu Glu Ser Asn Pro Ser Val Arg Glu Phe Val
305                 310                 315                 320
Leu Ser Lys Gly Asp Ala Gly Leu Arg Glu Ala Tyr Asp Ala Cys Val
                325                 330                 335
Lys Ala Leu Val Ser Leu Arg Ser Tyr His Leu Gln Ile Val Thr Lys
            340                 345                 350
Tyr Ile Leu Ile Pro Ala Ser Gln Gln Pro Lys Glu Asn Lys Thr Ser
        355                 360                 365
Glu Asp Pro Ser Lys Leu Glu Ala Lys Gly Thr Gly Gly Thr Asp Leu
    370                 375                 380
Met Asn Phe Leu Lys Thr Val Arg Ser Thr Thr Glu Lys Ser Leu Leu
385                 390                 395                 400
Lys Glu Gly
```

SEQ ID NO: 14 (PD-L1)

```
Met Arg Ile Phe Ala Val Phe Ile Phe Met Thr Tyr Trp His Leu Leu
1               5                   10                  15
Asn Ala Phe Thr Val Thr Val Pro Lys Asp Leu Tyr Val Val Glu Tyr
            20                  25                  30
Gly Ser Asn Met Thr Ile Glu Cys Lys Phe Pro Val Glu Lys Gln Leu
        35                  40                  45
Asp Leu Ala Ala Leu Ile Val Tyr Trp Glu Met Glu Asp Lys Asn Ile
    50                  55                  60
Ile Gln Phe Val His Gly Glu Glu Asp Leu Lys Val Gln His Ser Ser
65                  70                  75                  80
Tyr Arg Gln Arg Ala Arg Leu Leu Lys Asp Gln Leu Ser Leu Gly Asn
                85                  90                  95
Ala Ala Leu Gln Ile Thr Asp Val Lys Leu Gln Asp Ala Gly Val Tyr
            100                 105                 110
Arg Cys Met Ile Ser Tyr Gly Gly Ala Asp Tyr Lys Arg Ile Thr Val
        115                 120                 125
Lys Val Asn Ala Pro Tyr Asn Lys Ile Asn Gln Arg Ile Leu Val Val
    130                 135                 140
Asp Pro Val Thr Ser Glu His Glu Leu Thr Cys Gln Ala Glu Gly Tyr
145                 150                 155                 160
Pro Lys Ala Glu Val Ile Trp Thr Ser Ser Asp His Gln Val Leu Ser
                165                 170                 175
Gly Lys Thr Thr Thr Thr Asn Ser Lys Arg Glu Glu Lys Leu Phe Asn
            180                 185                 190
Val Thr Ser Thr Leu Arg Ile Asn Thr Thr Thr Asn Glu Ile Phe Tyr
        195                 200                 205
Cys Thr Phe Arg Arg Leu Asp Pro Glu Glu Asn His Thr Ala Glu Leu
    210                 215                 220
Val Ile Pro Glu Leu Pro Leu Ala His Pro Pro Asn Glu Arg Thr His
225                 230                 235                 240
Leu Val Ile Leu Gly Ala Ile Leu Leu Cys Leu Gly Val Ala Leu Thr
                245                 250                 255
Phe Ile Phe Arg Leu Arg Lys Gly Arg Met Met Asp Val Lys Lys Cys
            260                 265                 270

Gly Ile Gln Asp Thr Asn Ser Lys Lys Gln Ser Asp Thr His Leu Glu
        275                 280                 285
Glu Thr
    290
```

## Further aspects of the invention

1. A method for the prevention or treatment of cancer in a subject, the method comprising administering to said subject:

(i) an immunotherapeutic composition comprising a component of an immune system checkpoint, or an immunogenic fragment of said component; and
(ii) an immunomodulatory agent which blocks or inhibits an immune system checkpoint, which checkpoint may be the same as, or different from, the checkpoint of which the composition of (i) comprises a component.

2. A method according to aspect 1, wherein at least one said checkpoint is selected from the following:

a) The interaction between IDO1 and its substrate;
b) The interaction between PD1 and PDL1 and/or PD1 and PDL2;
c) The interaction between CTLA4 and CD86 and/or CTLA4 and CD80;
d) The interaction between B7-H3 and/or B7-H4 and their respective ligands;
e) The interaction between HVEM and BTLA;

f) The interaction between GAL9 and TIM3;
g) The interaction between MHC class I or II and LAG3; and
h) The interaction between MHC class I or II and KIR

3. A method according to aspect 2, wherein:

(A) the composition of (i) comprises a component of checkpoint (a) or an immunogenic fragment thereof, and the agent of (ii) blocks or inhibits the same or a different checkpoint, optionally wherein the different checkpoint is checkpoint (b) or (c);
or
(B) the composition of (i) comprises a component of checkpoint (b) or an immunogenic fragment thereof, and the agent of (ii) blocks or inhibits the same or a different checkpoint, optionally wherein the different checkpoint is checkpoint (a) or (c).

4. A method according to any one of the preceding aspects wherein said component of said immune system checkpoint is IDOI (SEQ ID NO: 1) and said immunogenic fragment of IDO consists of up to 25 consecutive amino acids of the sequence of SEQ ID NO: 1, wherein said consecutive amino acids comprise the sequence of ALLEIASCL (SEQ ID NO: 2) or the sequence of DTLLKALLEIASCLEKALQVF (SEQ ID NO: 3).

5. A method according to aspect 4 wherein said immunogenic fragment comprises or consists of the sequence of ALLEIASCL (SEQ ID NO: 2) or the sequence of DTLLKALLEIASCLEKALQVF (SEQ ID NO: 3).

6. A method according to any one of aspects 1 to 3, wherein said component of said immune system checkpoint is PD-L1 (SEQ ID NO: 14) and said immunogenic fragment of PD-L1 consists of up to 25 consecutive amino acids of the sequence of SEQ ID NO: 14, wherein said consecutive amino acids comprise the sequence of any one of SEQ ID NOs: 15 to 31, preferably of any one of SEQ ID NOs: 15, 25 or 28.

7. A method according to any one of the preceding aspects wherein said immunomodulatory agent is an antibody or small molecule inhibitor (SMI) which binds to a component of a said immune system checkpoint.

8. A method according to aspect 7, wherein said agent is a small molecule inhibitor of IDOl, optionally wherein said inhibitor is Epacadostat (INCB24360), Indoximod, GDC-0919 (NLG919) or F001287, or wherein said agent is an antibody which binds to CTLA4 or PD1, optionally wherein said antibody which binds to CTLA4 is ipilimumab and said antibody which binds to PD1 is pembrolizumab.

9. A method according to any one of the preceding aspects wherein said composition of (i) includes an adjuvant or carrier, optionally wherein said adjuvant is selected from a bacterial DNA adjuvant, an oil/surfactant adjuvant, a viral dsRNA adjuvant, an imidazoquinoline and GM-CSF.

10. A method according to aspect 9 wherein said adjuvant is a Montanide ISA adjuvant, optionally selected from Montanide ISA 51 or Montanide ISA 720.

11. An immunotherapeutic composition for use in a method for the prevention or treatment of cancer in a subject, the immunotherapeutic composition comprising a component of an immune system checkpoint or an immunogenic fragment thereof, and the method comprising administering to said subject:

(i) said immunotherapeutic composition; and
(ii) an immunomodulatory agent.

12. Use of an immunotherapeutic composition in the manufacture of a medicament for the prevention or treatment of cancer in a subject, the immunotherapeutic composition comprising a component of an immune system checkpoint or an immunogenic fragment thereof, which is formulated for administration before, concurrently with, and/or after an immunomodulatory agent.

13. A kit comprising:

(i) An immunotherapeutic composition comprising a component of an immune system checkpoint or an immunogenic fragment thereof; and/or

(ii) An immunomodulatory agent;

optionally wherein (i) and (ii) are provided in separate sealed containers.

14. An immunotherapeutic composition comprising an adjuvant and an immunogenic fragment of IDO which consists of up to 25 consecutive amino acids of the sequence of SEQ ID NO: 1, wherein said consecutive amino acids comprise the sequence of ALLEIASCL (SEQ ID NO: 2) or the sequence of DTLLKALLEIASCLEKALQVF (SEQ ID NO: 3).

15. An immunotherapeutic composition according to aspect 14, wherein the immunogenic fragment of IDO consists of the sequence of DTLLKALLEIASCLEKALQVF (SEQ ID NO: 3)

16. An immunotherapeutic composition according to aspect 14 or 15 for use in a method for the prevention or treatment of cancer in a subject, optionally wherein said method is a method according to any one of aspects 1 to 10.

17. Use of an immunotherapeutic composition according to aspect 14 or 15 in the manufacture of a medicament for the prevention or treatment of cancer in a subject.

## Claims

1. An immunotherapeutic composition for use in a method for the prevention or treatment of cancer in a subject, the method comprising administering to said subject:

   (i) said immunotherapeutic composition, which comprises a component of an immune system checkpoint, or an immunogenic fragment of said component; and
   (ii) an immunomodulatory agent which blocks or inhibits an immune system checkpoint, which checkpoint may be the same as, or different from, the checkpoint of which the composition of (i) comprises a component.

2. The immunotherapeutic composition for use according to claim 1, wherein at least one said checkpoint is selected from the following:

   a) The interaction between IDO1 and its substrate;
   b) The interaction between PD1 and PDL1 and/or PD1 and PDL2;
   c) The interaction between CTLA4 and CD86 and/or CTLA4 and CD80;
   d) The interaction between B7-H3 and/or B7-H4 and their respective ligands;
   e) The interaction between HVEM and BTLA;
   f) The interaction between GAL9 and TIM3;
   g) The interaction between MHC class I or II and LAG3; and
   h) The interaction between MHC class I or II and KIR

3. The immunotherapeutic composition for use according to claim 2, wherein:

   (A) the composition of (i) comprises a component of checkpoint (a) or an immunogenic fragment thereof, and the agent of (ii) blocks or inhibits the same or a different checkpoint, optionally wherein the different checkpoint is checkpoint (b) or (c);
   or
   (B) the composition of (i) comprises a component of checkpoint (b) or an immunogenic fragment thereof, and the agent of (ii) blocks or inhibits the same or a different checkpoint, optionally wherein the different checkpoint is checkpoint (a) or (c).

4. The immunotherapeutic composition for use according to any one of the preceding claims wherein said component of said immune system checkpoint is IDO1 (SEQ ID NO: 1) and said immunogenic fragment of IDO consists of up to 25 consecutive amino acids of the sequence of SEQ ID NO: 1, wherein said consecutive amino acids comprise the sequence of ALLEIASCL (SEQ ID NO: 2) or the sequence of DTLLKALLEIASCLEKALQVF (SEQ ID NO: 3).

5. The immunotherapeutic composition for use according to claim 4 wherein said immunogenic fragment comprises or consists of the sequence of ALLEIASCL (SEQ ID NO: 2) or the sequence of DTLLKALLEIASCLEKALQVF (SEQ ID NO: 3).

**6.** The immunotherapeutic composition for use according to any one of claims 1 to 3, wherein said component of said immune system checkpoint is PD-L1 (SEQ ID NO: 14) and said immunogenic fragment of PD-L1 consists of up to 25 consecutive amino acids of the sequence of SEQ ID NO: 14, wherein said consecutive amino acids comprise the sequence of any one of SEQ ID NOs: 15 to 31, preferably of any one of SEQ ID NOs: 15, 25 or 28.

**7.** The immunotherapeutic composition for use according to any one of the preceding claims wherein said immunomodulatory agent is an antibody or small molecule inhibitor (SMI) which binds to a component of a said immune system checkpoint.

**8.** The immunotherapeutic composition for use according to claim 7, wherein said agent is a small molecule inhibitor of IDO1, optionally wherein said inhibitor is Epacadostat (INCB24360), Indoximod, GDC-0919 (NLG919) or F001287, or wherein said agent is an antibody which binds to CTLA4 or PD1, optionally wherein said antibody which binds to CTLA4 is ipilimumab and said antibody which binds to PD1 is pembrolizumab.

**9.** The immunotherapeutic composition for use according to any one of the preceding claims wherein said composition of (i) includes an adjuvant or carrier, optionally wherein said adjuvant is selected from a bacterial DNA adjuvant, an oil/surfactant adjuvant, a viral dsRNA adjuvant, an imidazoquinoline and GM-CSF.

**10.** The immunotherapeutic composition for use according to claim 9 wherein said adjuvant is a Montanide ISA adjuvant, optionally selected from Montanide ISA 51 or Montanide ISA 720.

**11.** A kit comprising:

(i) An immunotherapeutic composition comprising a component of an immune system checkpoint or an immunogenic fragment thereof; and/or
(ii) An immunomodulatory agent;

optionally wherein (i) and (ii) are provided in separate sealed containers.

**12.** An immunotherapeutic composition comprising an adjuvant and an immunogenic fragment of IDO which consists of up to 25 consecutive amino acids of the sequence of SEQ ID NO: 1, wherein said consecutive amino acids comprise the sequence of ALLEIASCL (SEQ ID NO: 2) or the sequence of DTLLKALLEIASCLEKALQVF (SEQ ID NO: 3).

**13.** An immunotherapeutic composition according to claim 12, wherein the immunogenic fragment of IDO consists of the sequence of DTLLKALLEIASCLEKALQVF (SEQ ID NO: 3).

# Figure 1

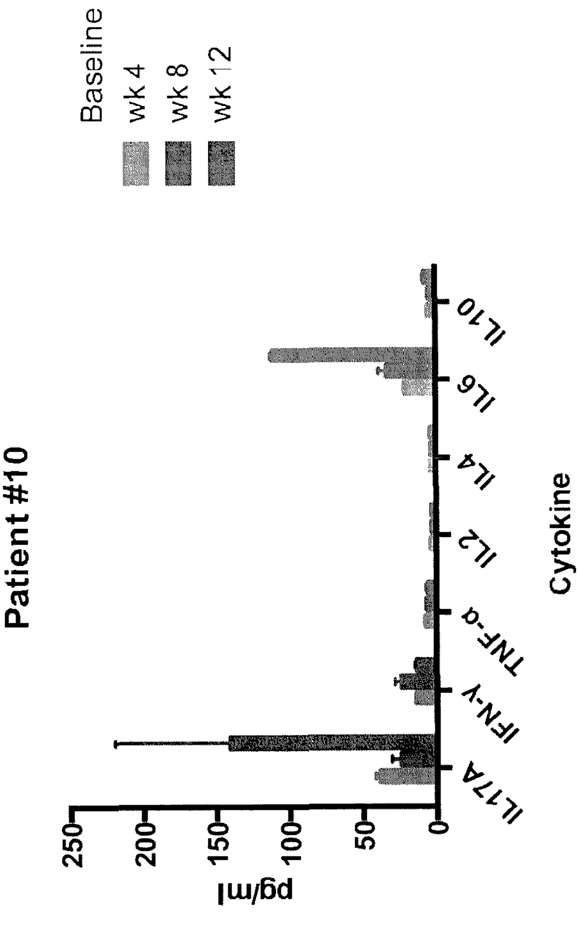
Patient #10
pg/ml
250
200
150
100
50
0
IL17A
IFN-γ
TNF-α
IL2
IL4
IL6
IL10
Cytokine
Baseline
wk 4
wk 8
wk 12

# Figure 2a

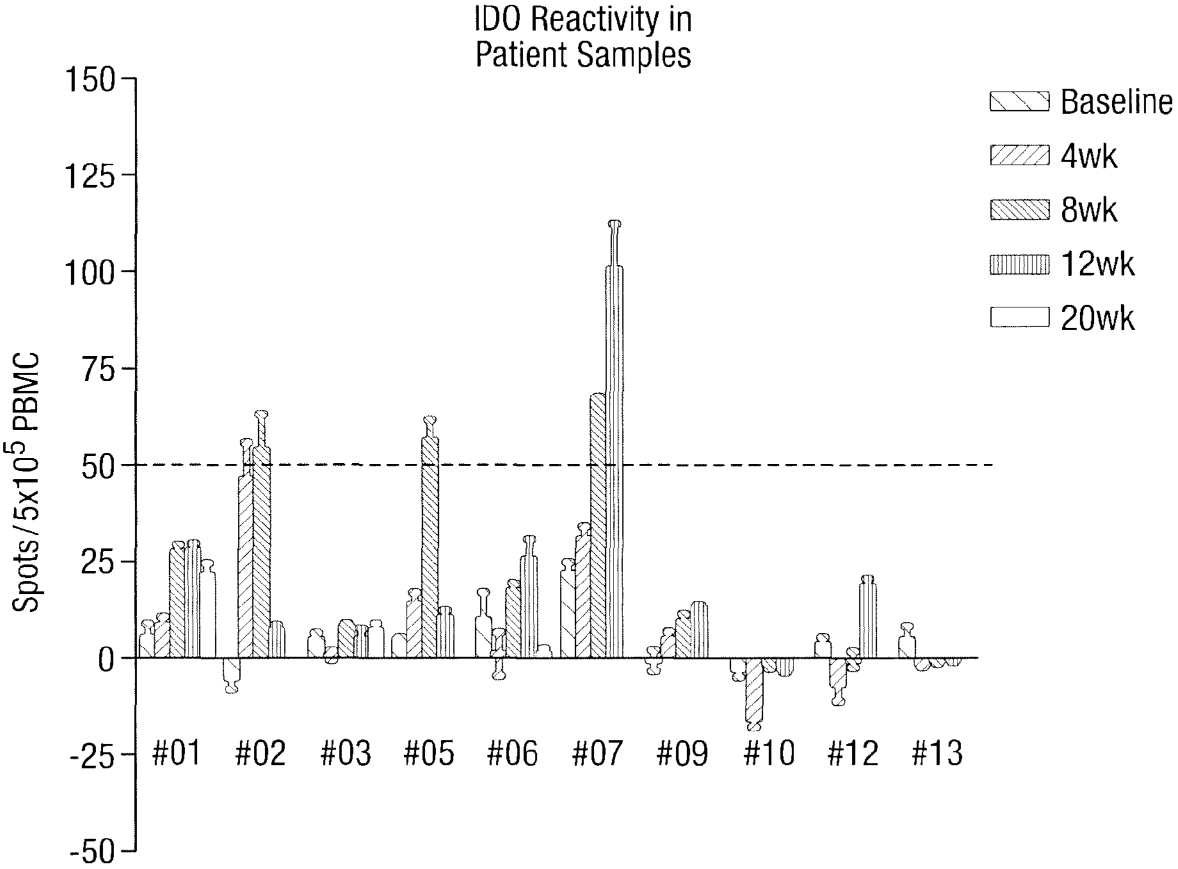


# Figure 2b

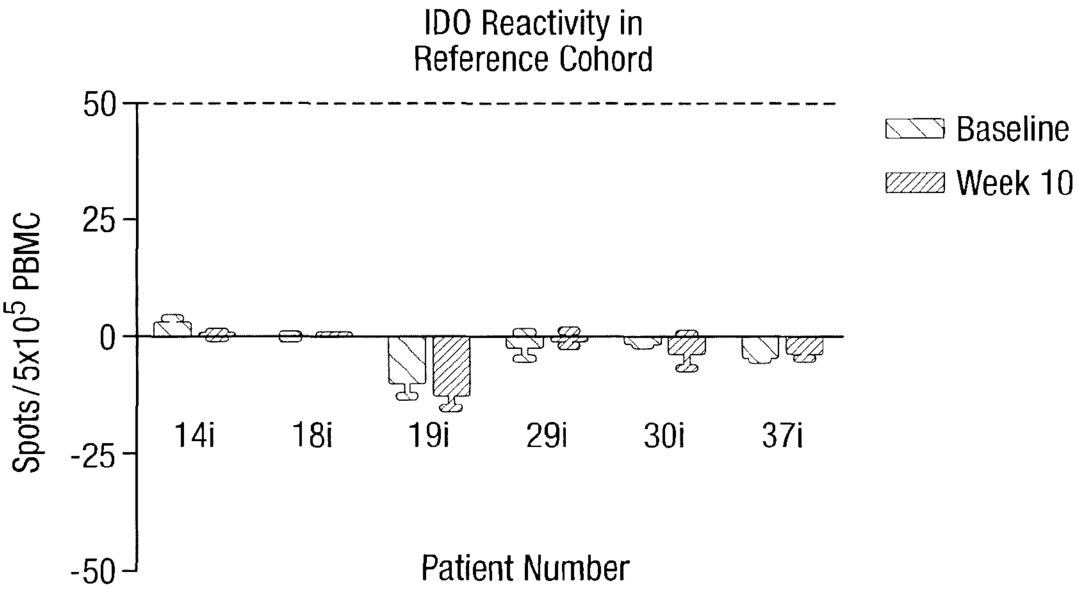

# Figure 2c

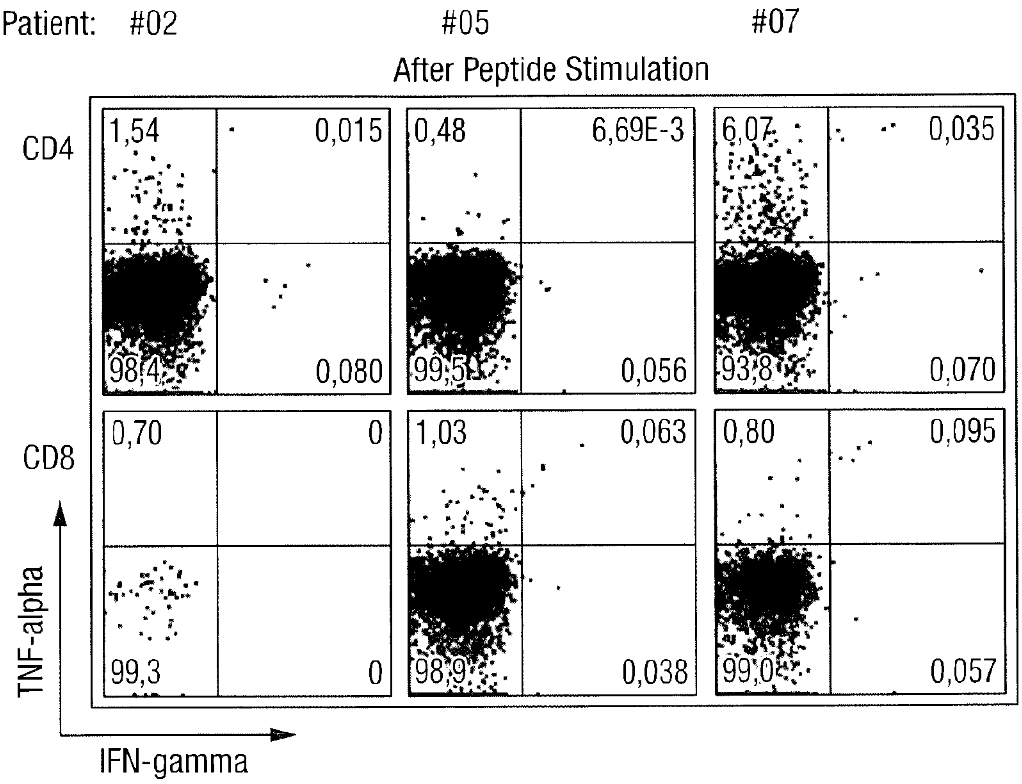


# Figure 2d

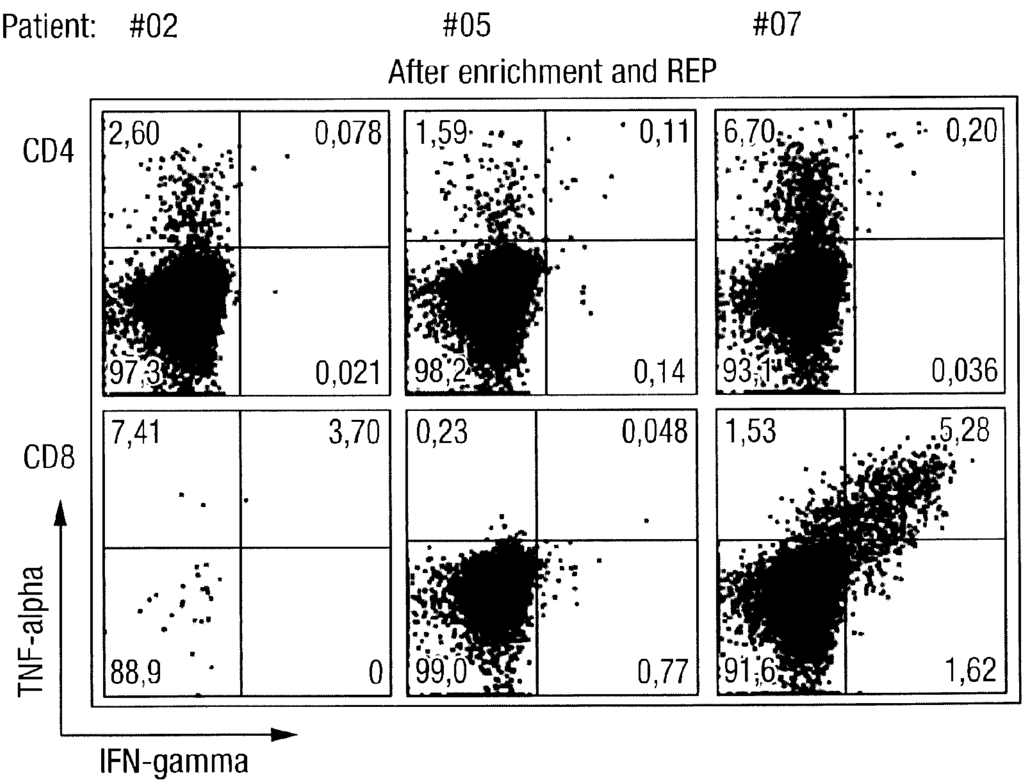

Figure 3a

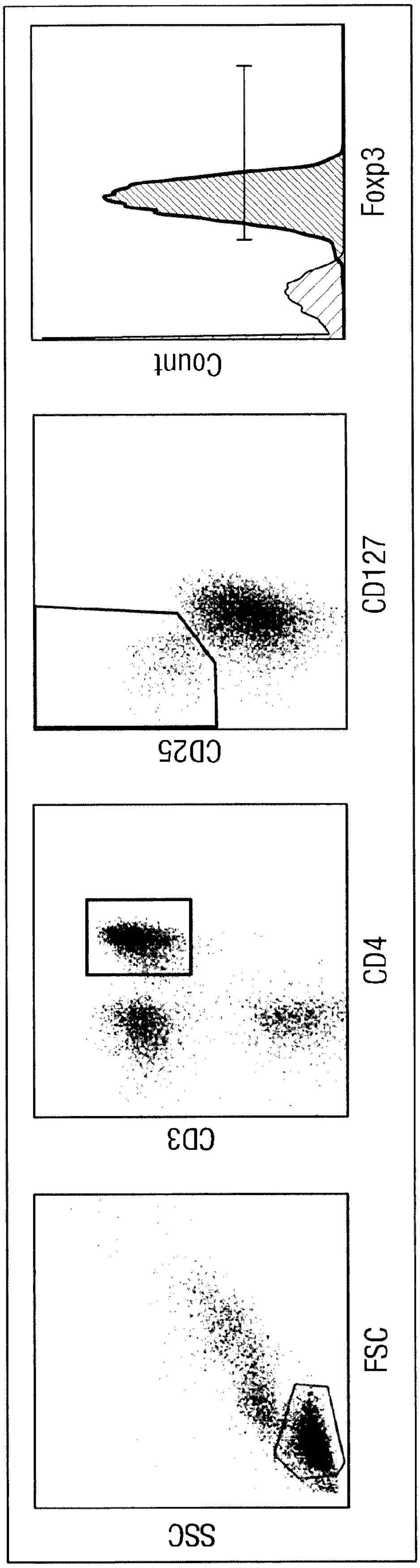


Figure 3b

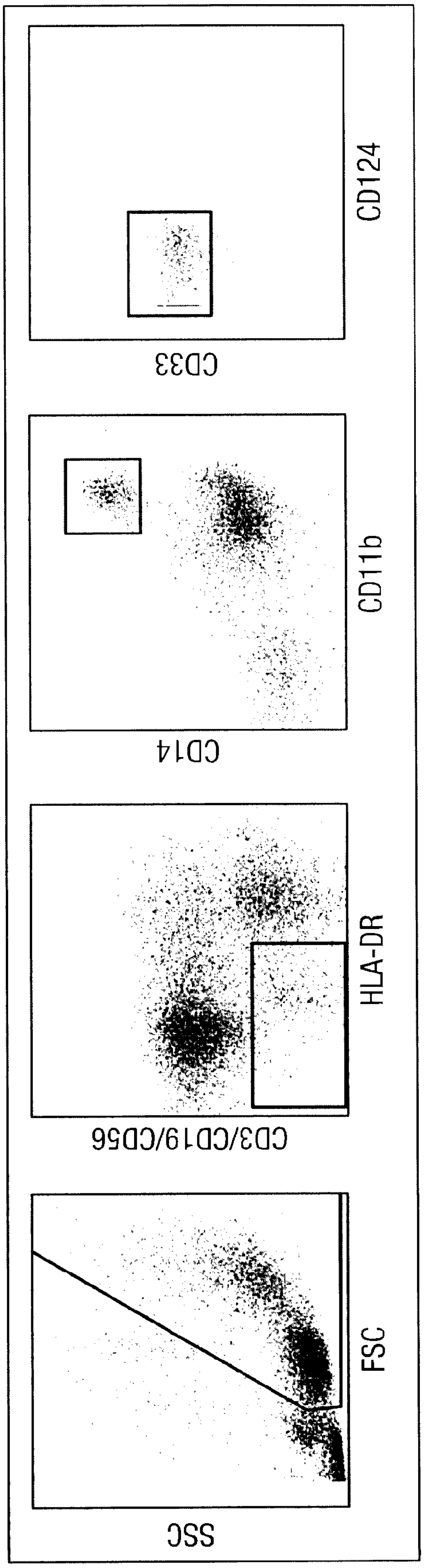

Figure 3c

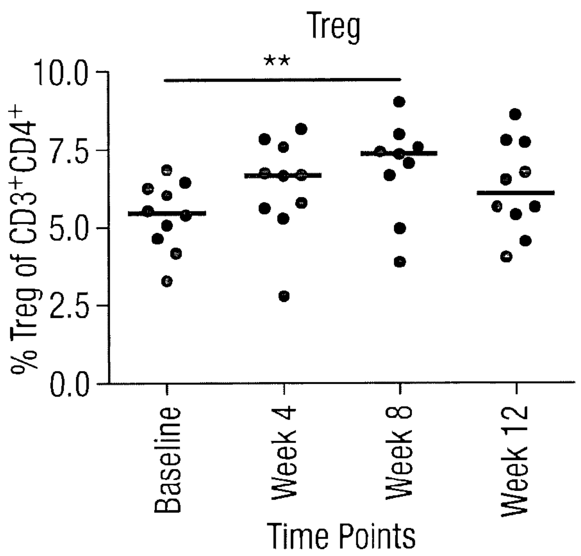


Figure 3d

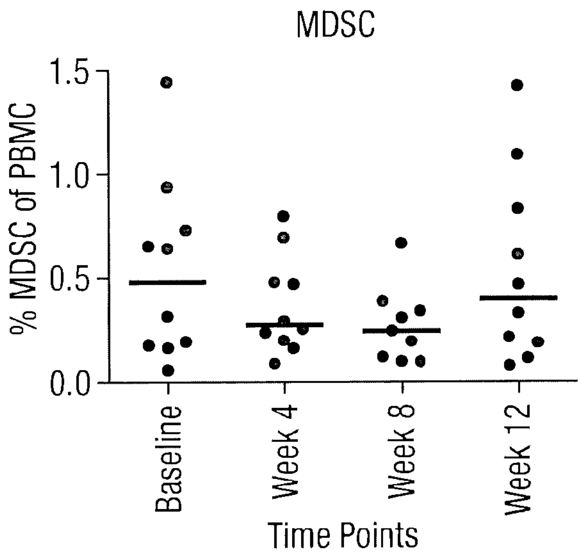


Figure 3e

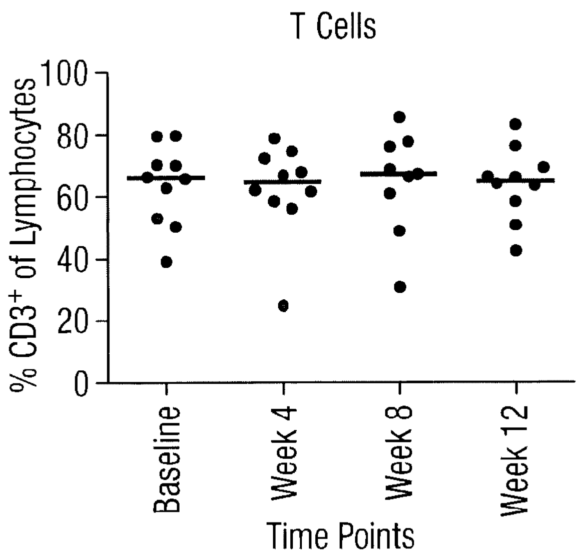


Figure 3f

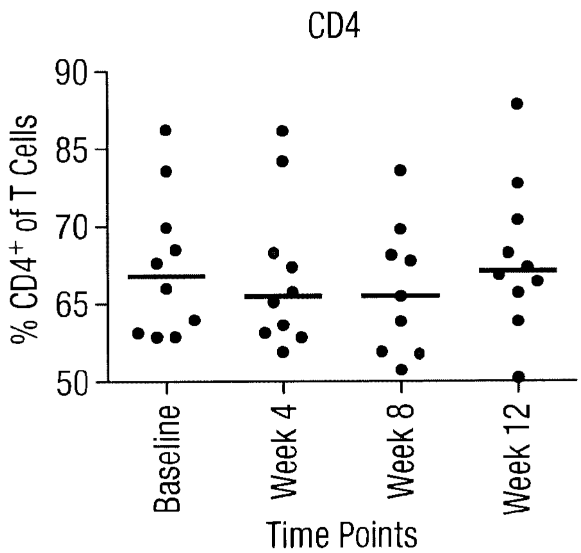

Figure 4

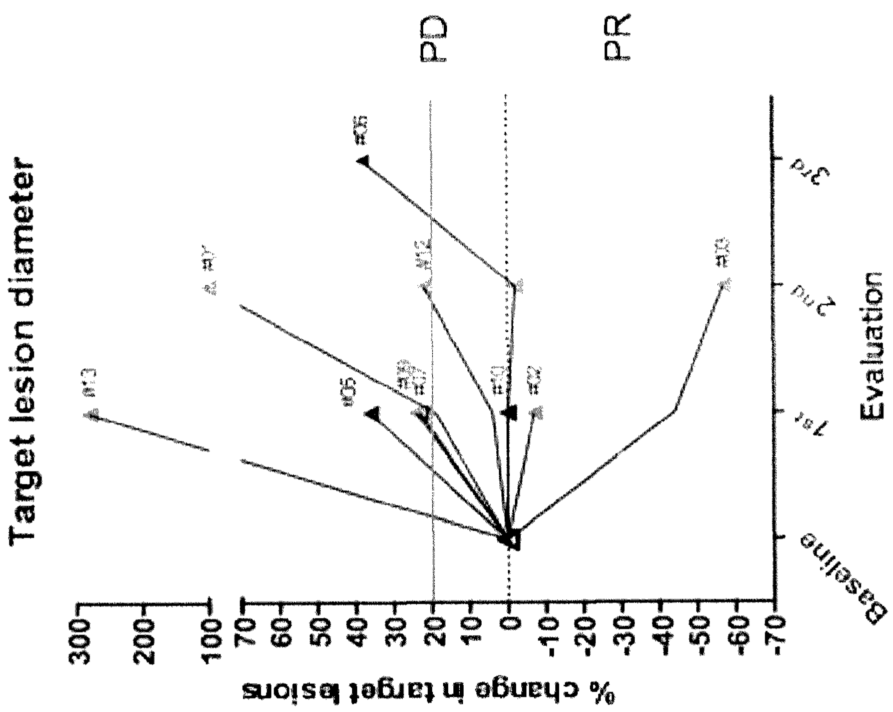
Target lesion diameter
% change in target lesions
300
200
100
70
60
50
40
30
20
10
0
-10
-20
-30
-40
-50
-60
-70
PD
PR
Baseline
1st
2nd
3rd
Evaluation

Figure 5a

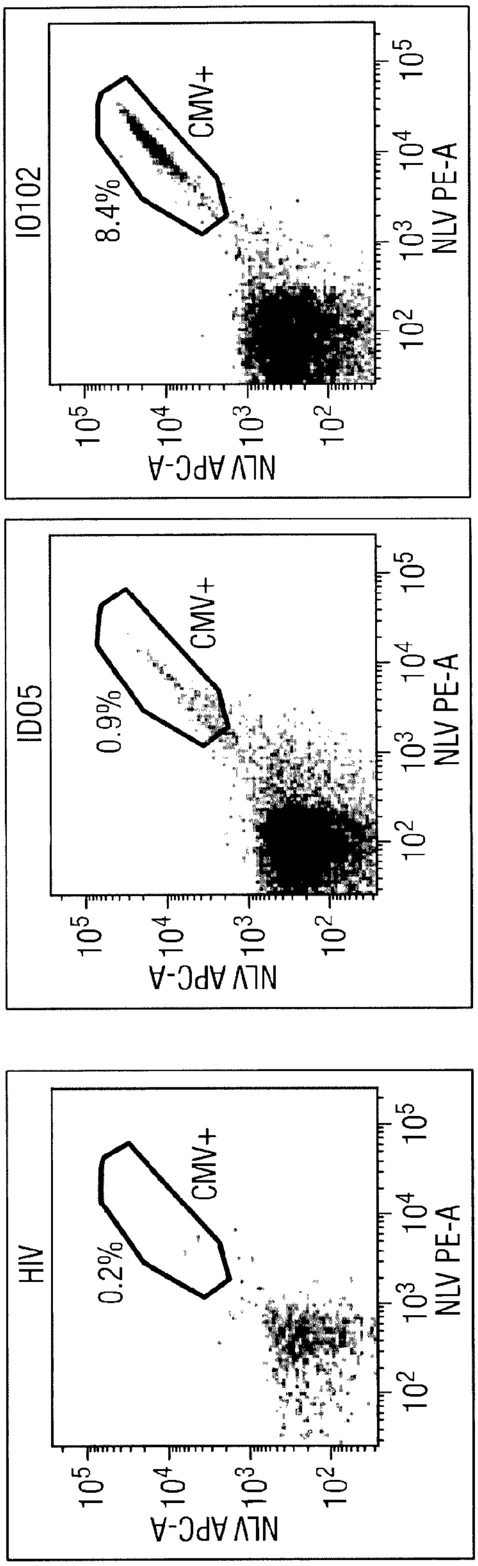


Figure 5b

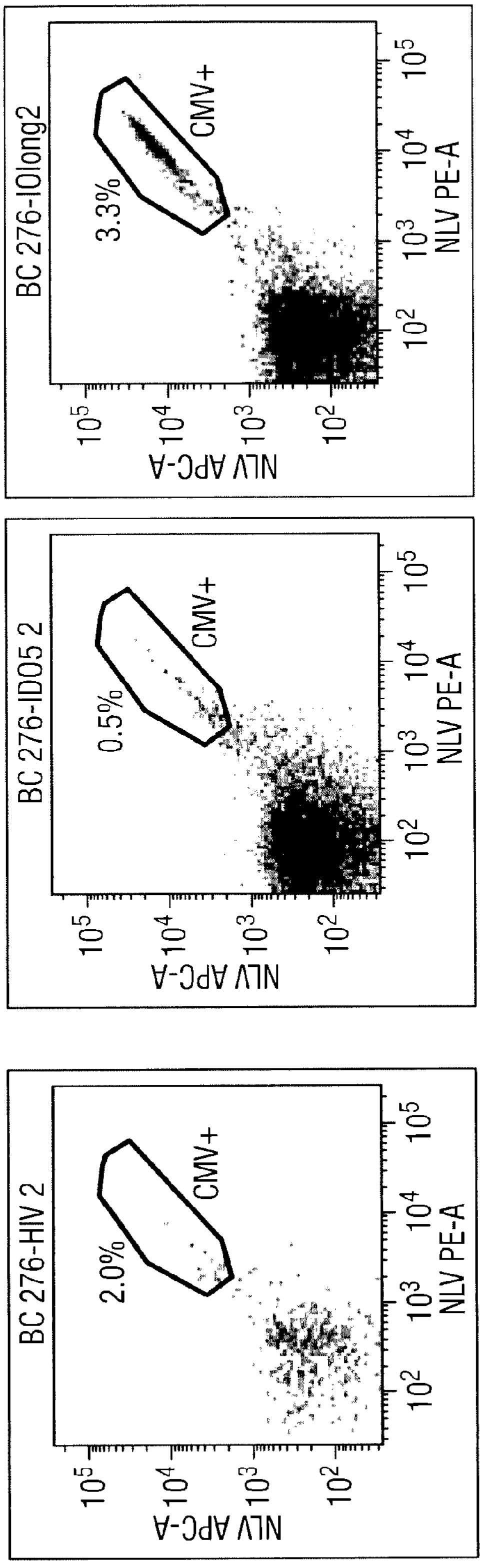

Figure 6

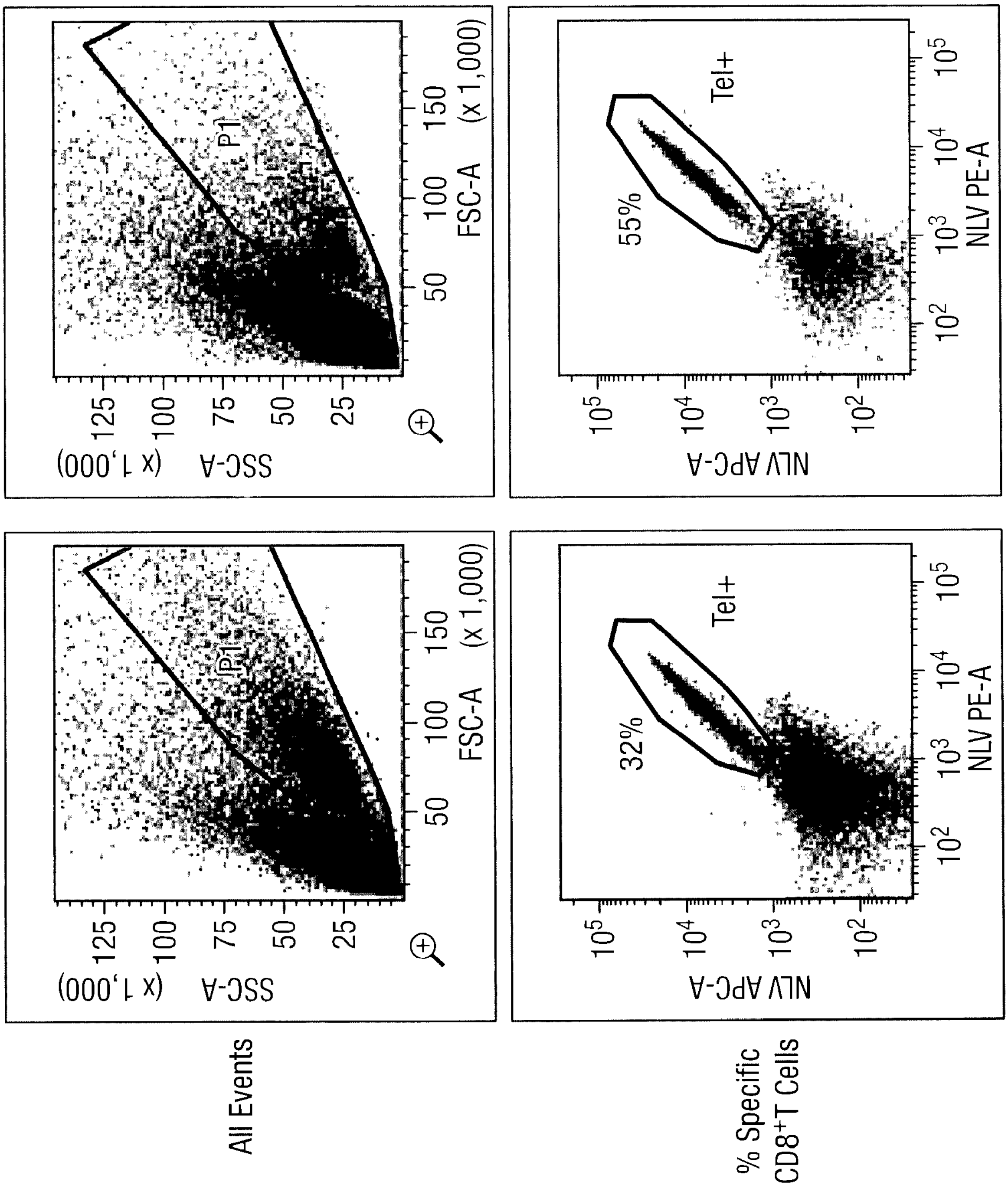
IDO SMI (1-MT)
IO102 + IDO SMI (1-MT)
All Events
% Specific
CD8+T Cells
SSC-A (x 1,000)
FSC-A (x 1,000)
P1
NLV APC-A
NLV PE-A
Tel+
32%
55%

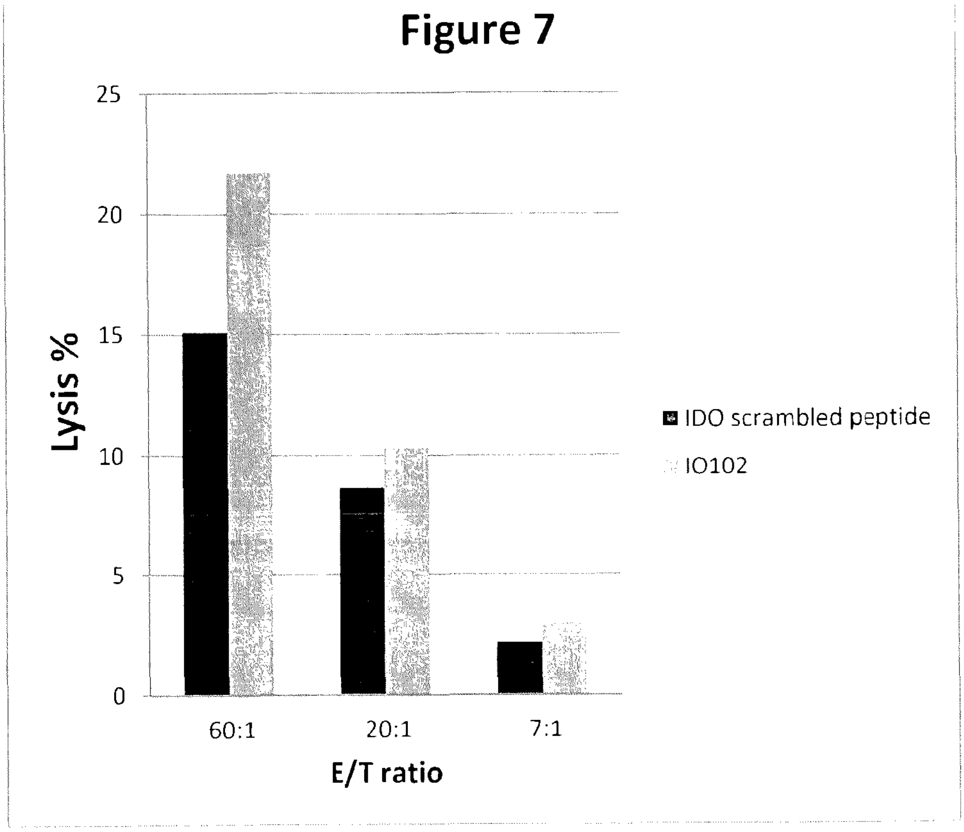
Figure 7
25
20
15
10
5
0
Lysis %
IDO scrambled peptide
IO102
60:1
20:1
7:1
E/T ratio

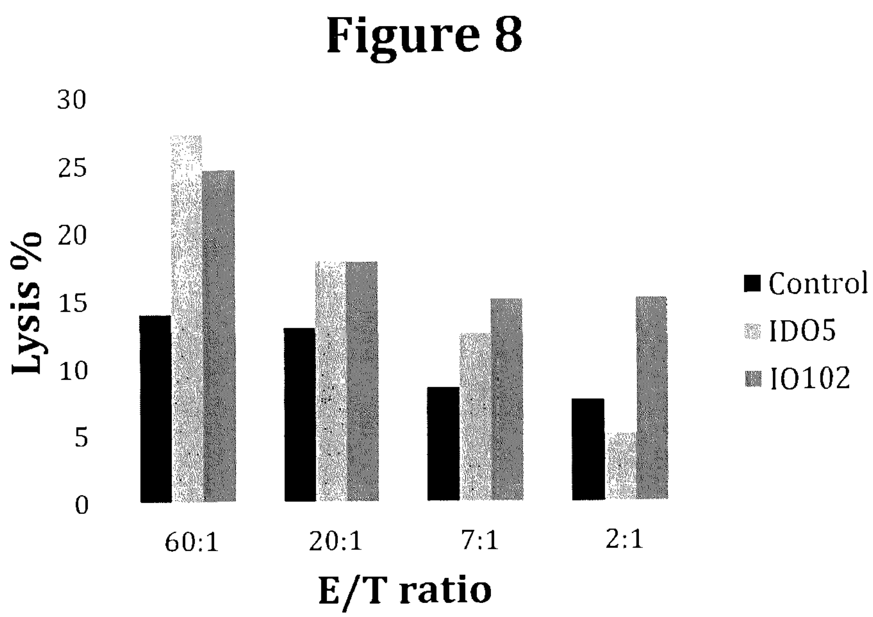
Figure 8
30
25
20
15
10
5
0
Lysis %
Control
IDO5
IO102
60:1
20:1
7:1
2:1
E/T ratio

Figure 9

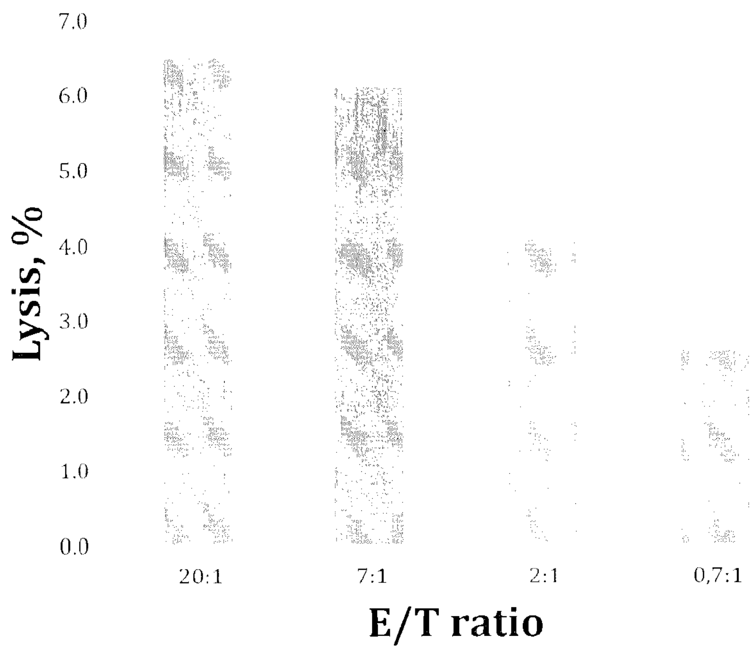

# Figure 10

A)

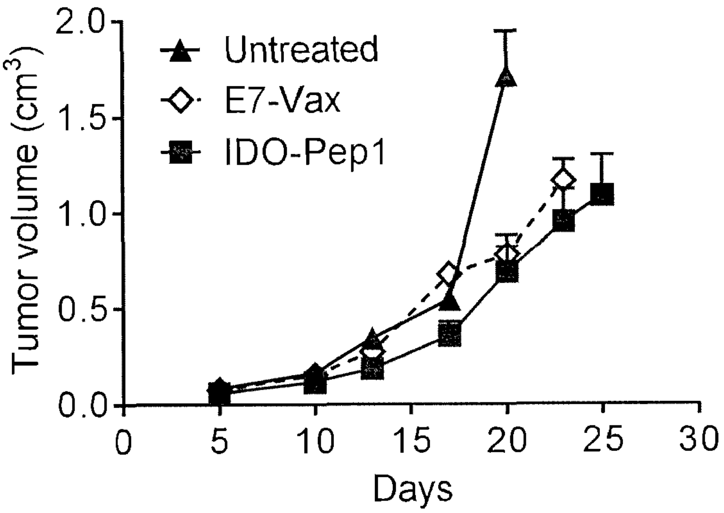
Tumor volume (cm$^3$)
2.0
1.5
1.0
0.5
0.0
Untreated
E7-Vax
IDO-Pep1
0
5
10
15
20
25
30
Days

B)

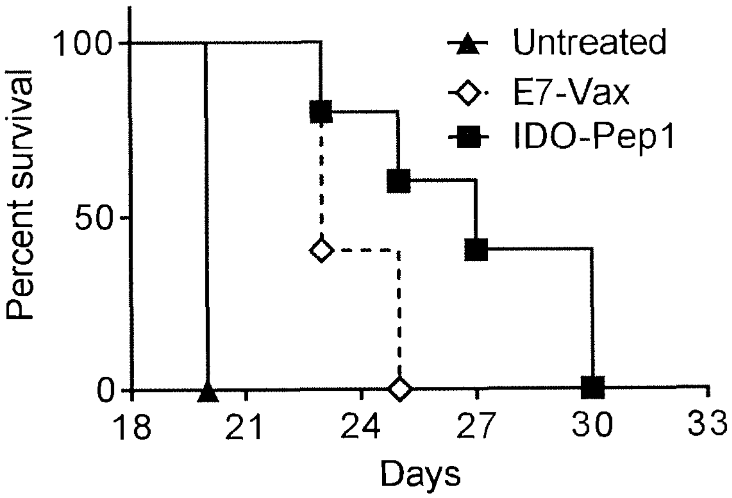
Percent survival
100
50
0
Untreated
E7-Vax
IDO-Pep1
18
21
24
27
30
33
Days

# Figure 11

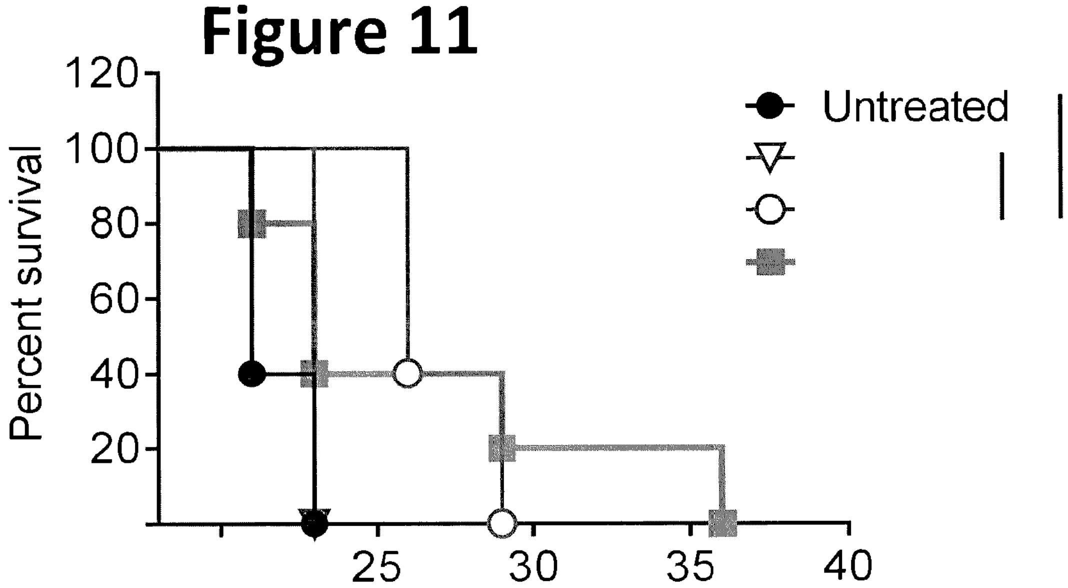


# Figure 12

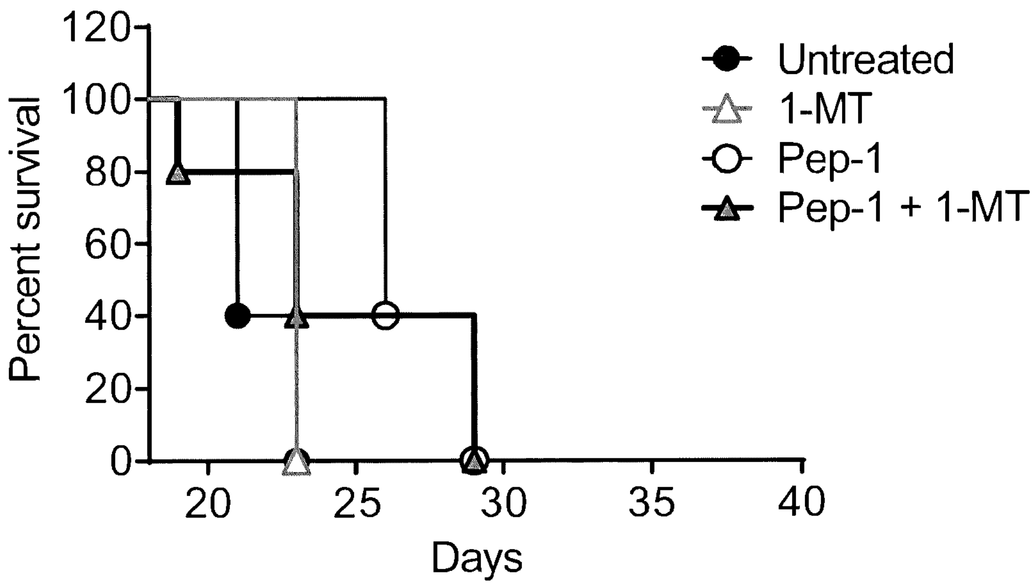

# Figure 13

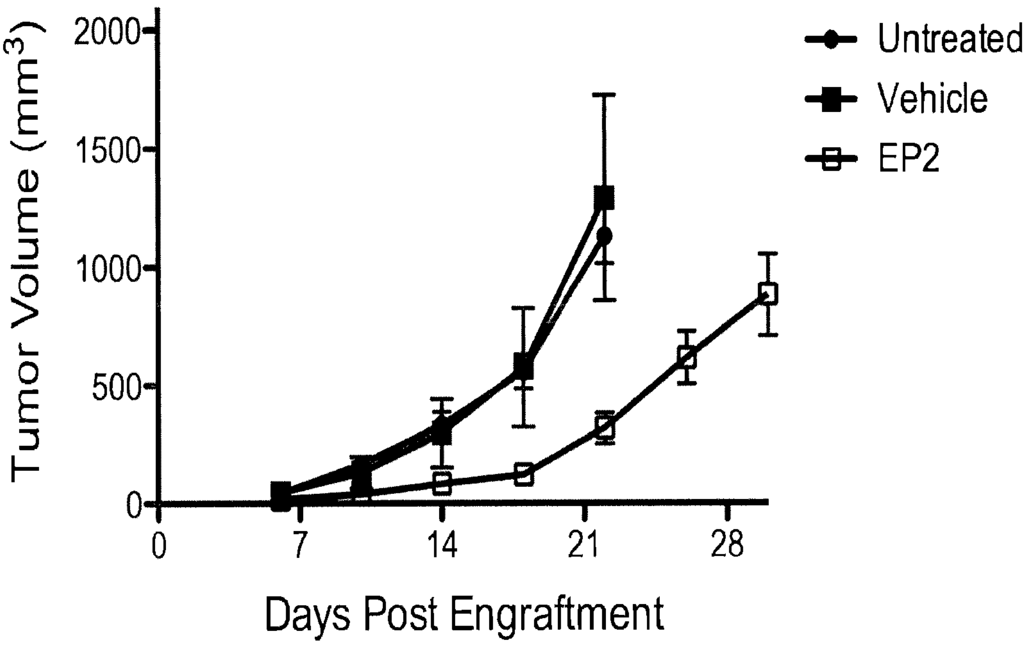
Tumor Volume (mm3)
2000
1500
1000
500
0
0
7
14
21
28
Days Post Engraftment
Untreated
Vehicle
EP2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009143843 A
- WO 2013056716 A
- US 58767 A
- US 5554372 A
- WO 2014207063 A


### Non-patent literature cited in the description


- **ANDERSEN ; SVANE.** *Oncoimmunology,* 2015, vol. 4 (1), e983770
- **IVERSEN et al.** *Clin Cancer Res,* 2014, vol. 20 (1), 221-32
- **GODING.** Monoclonal Antibodies: Principles & Practice. 1986, 61-63
- Remington's Pharmaceutical Sciences. Mack Pub. Co, 1991
- **JEFFERY et al.** *Pharm. Res.,* 1993, vol. 10, 362-368